# EUROPEAN PATENT APPLICATION

(11) **EP 4 678 224 A1**
(43) Date of publication of application: **14.01.2026**
(21) Application number: 25188656.0
(22) Date of filing: 10.07.2025
(51) Int. Cl.: A61N 1/375, A61N 1/362

(54) **PACEMAKERS WITH INTEGRATED LEAD AND DELIVERY SYSTEMS AND METHODS THEREFOR**

(30) Priority: 10.07.2024 US 202463669443 P; 18.04.2025 US 202563791014 P
(71) Applicant: Pacesetter, Inc., Sylmar, CA 91342 (US)
(72) Inventor: Alleman, Wesley, Santa Clarita, Los Angeles, CA 91350 (US); Mouchawar, Gabriel, Valencia, Los Angeles, CA 91355 (US); Ganz, Leonard, Marina del Rey, Los Angeles, CA 90292 (US); Shaw, Robert, Castaic, Los Angeles, CA 91384 (US); Victorine, Keith, Valencia, Los Angeles, CA 91355 (US); Dadashian, Ashgar, Porter Ranch, CA 91326 (US); Schmid, Logan, San Diego, CA 92128 (US); Fishler, Matthew, Scotts Valley, CA 95066 (US); Charan, Vish, Valencia, CA 91381 (US); Reyes, Efrain, San Fernando, CA 91340 (US)
(74) Representative: Barker Brettell Sweden AB

(57) **Abstract**

A pacemaker device (400) including a pacemaker portion (402) and an integrated connector (2904). The pacemaker device (400) includes a housing assembly (3316, 4300) defining a plurality of housings (3302, 3304, 4302, 4304, 4306, 4308) for containing pacing electronics, battery material and a connector (2904, 4904). The connector (2904, 4904) is enclosed within the housing (3304, 4304, 4308) and is configured to receive a lead (3202) which is electrically connectable with the pacing electronics. The pacemaker device (400) may include a leadless pacemaker (102) inserted in the housing assembly (3316) configured to convert the leadless pacemaker (102) into a leaded configuration. The leadless pacemaker (102) may be removably attached from the pacemaker device (400) and replaced with another leadless pacemaker (102).

## Description

### TECHNICAL FIELD

This disclosure relates generally to implantable pacemakers or biostimulators. More specifically, this disclosure relates to pacemaker systems with integrated leads or converted leads.

### BACKGROUND

Current epicardial pacemaker systems follow the same convention as transvenous systems, with an implantable pulse generator (IPG) connected to a cardiac lead that is then attached to the epicardial surface of the patient's heart. In this convention, the IPG is typically placed under the rectus abdominis muscle in the abdomen due to the size of the device, with the leads crossing the abdominal musculature. This creates a high risk of conductor fracture. Other IPG placement options intended to air in lead survivability or longevity, such as subxiphoid or retrocostal, are limed, for example, both in terms of the size of the patient and/or the size of the IPG itself.

Pediatric patients are almost exclusively limited to abdominal IPG placement. IPGs used in pediatric application are therefore at high risk of lead fracture before the patient has grown to a large enough size where a transvenous or leadless system may become an option. Additionally, due to the size of most IPGs available on the market, implantation of any IPG device connected to a lead is particularly challenging for neonates, as is lead management of these patients. For example, to compensate for rapid growth of the neonatal patient, excessive lead slack must be implanted and managed. Excessive slack creates additional risk factors, such as cardiac strangulation or greater risk of acute bending situations that have been associated with lead failure.

Additionally, current leaded pacemaker systems are decreasing in production as the parts required are commercially phased out. Pediatric patients can be at a higher risk of negative outcomes with implanted leadless pacemakers. This is due to smaller size of the femoral vessels which leadless pacemakers can cause occlusions or tears in the pediatric patients. With the phase out of traditional pediatric leaded pacemakers, a need exists to provide a leaded pacemaker before the patient has grown large enough in size where a transvenous or leadless system may become an option.

Accordingly, a need exists for a pacemaker system that addresses and overcomes the above-described challenges and increases access to life sustaining pacing technology for a highly vulnerable patient population.

### SUMMARY

In one aspect, a cardiac pacemaker device includes a pacemaker portion and a lead integrated with the pacemaker portion. The pacemaker portion includes a housing defining a cavity for containing pacing electronics and battery material. The distal extension is coupled to the housing and includes a lead electrically connectable with the pacing electronics and extending distally from the housing to a distal end of the distal extension, a fixation element positioned at the distal end of the distal extension, the fixation element being configured for installation within an epicardium of a patient's heart, and an electrode electrically connectable with the lead and configured to sense and/or deliver electrical signals at the patient's heart.

In another aspect, a device assembly includes a first housing and a second housing. The first housing contains pacing electronic and battery material. The second housing defining a cavity for containing a connector. The connector is coupled to the device assembly operable to convert the leadless pacemaker into a leaded pacemaker and includes a lead electrically connectable with the pacing electronics and extending distally from the housing to a distal end of the distal extension, a fixation element positioned at the distal end of the distal extension, the fixation element being configured for installation within an epicardium of a patient's heart, and an electrode electrically connectable with the lead and configured to sense and/or deliver electrical signals at the patient's heart.

In another aspect, an adaptor includes a first housing, a second housing, a third housing, and a fourth housing. The first housing defining a first cavity and a third housing defining a third cavity wherein the first housing and the third housing are each sized suitably to receive pacing electronics and battery material. The second housing defining a second cavity and a fourth housing defining a fourth cavity wherein the second housing and the fourth housing are sized suitably to receive a connector. The connector is coupled to the pacemaker portions operable to convert the leadless pacemakers into a leaded pacemakers and includes a lead electrically connectable with the pacing electronics and extending distally from the adaptor to a distal end of the distal extension, a fixation element positioned at the distal end of the distal extension, the fixation element being configured for installation within an a patient's heart, and an electrode electrically connectable with the lead and configured to sense and/or deliver electrical signals at the patient's heart

### BRIEF DESCRIPTION OF THE DRAWINGS

Aspects of the present disclosure are set forth with particularity in the claims that follow. A better understanding of the features and advantages of the present disclosure will be obtained by reference to the following detailed description that sets forth illustrative embodiments, in which the principles of the present disclosure are utilized, and the accompanying drawings of which:
FIG. 1 is a side view of an example cardiac pacemaker device.
FIG. 2 is a diagrammatic medial-lateral cross section of a patient heart illustrating example implantation of pacemaker devices in the patient heart.
FIG. 3 is a perspective view of an embodiment of a pacemaker device with a proximal pacemaker portion and integrated distal lead in accordance with the present disclosure.
FIG. 4 is a magnified view of a distal end of the pacemaker device of FIG. 3.
FIG. 5 is a magnified view of the distal end of the pacemaker device of FIG. 3 in accordance with an alternative embodiment.
FIGS. 6 and 7 are perspective views of an alternative embodiment of a pacemaker device with a proximal pacemaker portion and integrated distal lead in accordance with the present disclosure.
FIGS. 8-11 are magnified views of the distal end of the pacemaker device of FIGS. 6 and 7 in accordance with various embodiments of the present disclosure.
FIG. 12 is a magnified view of a proximal end of the pacemaker portion of the devices of FIGS. 3, 6, and 7.
FIG. 13 is a magnified view of a distal end of the pacemaker portion of the devices of FIGS. 3, 6, and 7.
FIG. 14 is a perspective view of an example delivery tool for the pacemaker devices of the present disclosure.
FIG. 15 is a cross-section of the delivery tool shown in FIG. 14.
FIG. 16 is a magnified view of a distal end of a pacemaker device with features to rotatably fix the device relative to the delivery tool of FIGS. 14 and 15.
FIGS. 17 and 18 are a magnified view and cross-section, respectively, showing engagement between features of the pacemaker device and the delivery tool to rotatably fix the pacemaker device.
FIGS. 19 and 20 are cross-sections depicting a trigger-release mechanism of the delivery tool used to selectively retain and release the pacemaker device during delivery.
FIG. 21 is a perspective view of a pacemaker device that includes a receptacle configured to enable replacing the device without replacing a pacing lead.
FIG. 22 is a cross-section of the receptacle of FIG. 21.
FIGS. 23 and 24 depict a pacemaker device in accordance with the present disclosure implanted within a patient's body using a subxiphoid approach with the pacemaker portion attachment to under side of the ribs.
FIG. 25 is a front view of an example conventional leaded pacemaker.
FIG. 26 is a side view of two example leadless cardiac pacemakers.
FIG. 27 depicts an example conventional leaded pacemaker and lead placement.
FIG. 28 depicts placement of an example conventional leadless pacemaker.
FIG. 29A depicts an example of a connector.
FIG. 29B depicts a device assembly including a pacemaker device and a connector.
FIGS. 30 and 31 are a front view and a side view, respectively, of an example of a device assembly including a pacemaker device, a connector and a housing assembly enclosing the connector and partially enclosing the pacemaker device.
FIG. 32 is a side view of the device assembly and housing assembly of FIGS. 30 and 31 with a distal lead in accordance with the present disclosure.
FIGS. 33-35 are a perspective view, a front view and a side view, respectively, of an example of a device assembly including a pacemaker device, a connector, and a housing assembly enclosing the connector and partially enclosing the pacemaker device.
FIG. 36 is a perspective view of another example of a device assembly including a pacemaker device, a connector, and a housing assembly enclosing the connector and partially enclosing the pacemaker device in an in-line configuration.
FIGS. 37 and 38 are perspective views of an example of a unipolar adaptor configured to receive a pacemaker device and a connector.
FIG. 39 and 40 are perspective views of an example of a bipolar adaptor configured to receive a pacemaker device and a connector.
FIG. 41 is a perspective view of an alternative example of a unipolar adaptor configured to receive two pacemaker devices and two connectors.
FIG. 42 is a perspective view of an alternative example of a bipolar adaptor configured to receive two pacemaker devices and two connectors.
FIG. 43 is a perspective view of another alternative example of a bipolar adaptor having wired communications capabilities and configured to receive two pacemaker devices and two connectors.
FIG. 44 is a perspective view of another example of an adaptor having a transversal opening and configured to receive a pacemaker device and a connector.
FIG. 45 is a perspective view of another example of a bipolar adaptor having a transversal opening and configured to receive a pacemaker device and a connector.
FIGS. 46-48 are a perspective view, a side view and a magnified side view, respectively, of an example of two device assemblies, each including a pacemaker device and a connector.
FIG. 49A is a perspective view of an example device assembly including a pacemaker device, a connector, and a housing assembly enclosing the pacemaker device and the connector.
FIG. 49B is a perspective view of the device assembly of FIG. 49A including an expanded view of a septum.

Corresponding and/or like reference numerals used throughout the drawings indicate corresponding and/or like features and elements.

### DETAILED DESCRIPTION

The embodiments described herein relate to pacemaker systems and methods that leverage leadless pacemaker technology to create a pacing device suitable for use in pediatric (e.g., neonatal) patients, which may be implanted on the epicardium, thereby increasing access to life sustaining pacing technology for a highly vulnerable patient population. This approach may reduce the risk of lead fracture and simplify management and help bridge transition from epicardial to transvenous or leadless pacing systems when the patients are large enough for these approaches. In the embodiments described herein, a pacing device includes a proximal pacemaker portion and distal extension coupled to the proximal pacemaker portion, the distal extension including a leaded conductor that extends distally from a distal end of the proximal pacemaker portion to a distal extension end including one or more fixation elements and one or more electrodes. The proximal pacemaker portion may be configured similar to a leadless pacemaker device, which provides a compact biostimulator volume which contains an energy source and circuitry, with the exception that a distal electrode of the leadless pacemaker is replaced with (or extended by) the distal extension. The leaded conductor extends from the proximal pacemaker portion to the fixation element(s), which is configured for implanting into the epicardium of the patient's heart, and the distal electrode(s), which is configured to sense and/or deliver electrical signals at the myocardium. In some embodiments, one or more of the fixation elements may also operate as an electrode, and are implanted into the epicardium at a sufficient depth to reach the myocardium for sensing and/or delivering electrical signals. The pacemaker is maintained in proximity to the heart, and compact size of the pacemaker enables a high degree of versatility with respect to the locations where the pacemaker is positioned.

Advantages provided by the embodiments described herein include, but are not limited to: 1) the device may be suitably sized for pediatric, and more specifically neonatal use; 2) the device may be sized to enable device placement in locations that may aid in lead survivability or longevity (subxiphoid, retrocostal, or potentially other implant locations within the rib cage), minimizing risk of lead fracture and need for additional surgical procedures; 3) the device may be placed within the rib cage, which maintains the device in proximity to the heart as the patient grows, minimizing need for excessive lead slack and slack management; 4) due to the size and form factor of the device, it may be used in conjunction with an implant tool and a thoracoscope (thoracoscopic camera) subxiphoid approach, thereby limiting or eliminating the need for epicardial placement via thoracotomy or other high-risk procedures.

Before beginning a detailed discussion of the pacemaker devices of the present disclosure, a general overview of an example leadless pacemaker is provided as follows.

### A. Overview of Intracardiac Leadless Pacemaker Devices

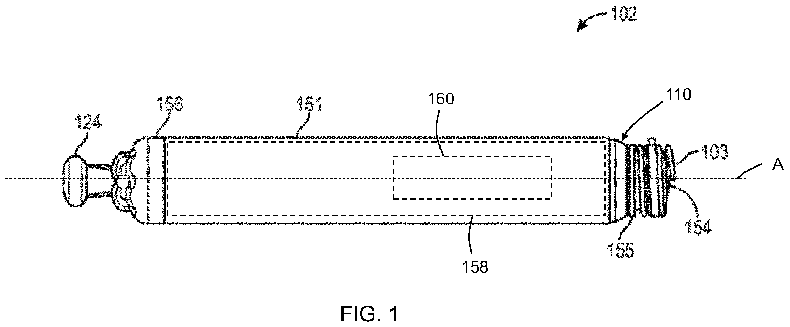

FIG. 1 illustrates an example intracardiac biostimulator, e.g., a leadless pacemaker 102, configured for implanting at an intracardial implant site (e.g., within an atrium or a ventricle of a patient's heart). The leadless pacemaker 102 can communicate by conducted communication, representing a substantial departure from conventional pacing systems. The leadless pacemaker 102 can perform cardiac pacing that has many of the advantages of conventional cardiac pacemakers while extending performance, functionality, and operating characteristics.

In some implementations, the leadless pacemaker 102 provides cardiac pacing without a pulse generator located in the pectoral region or abdomen, without an electrode-lead separate from the pulse generator, without a communication coil or antenna, and without an additional requirement of battery power for transmitted communication.

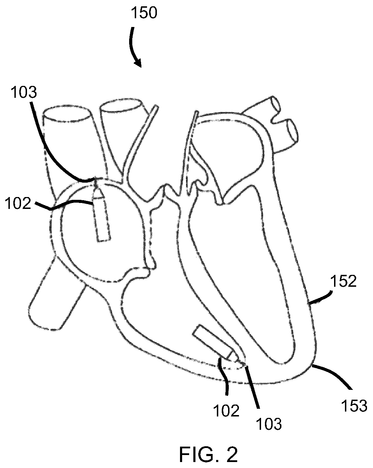

FIG. 2 illustrates an embodiment of a cardiac pacing system 150 configured to attain these characteristics. The cardiac pacing system 150 includes one or more leadless cardiac pacemakers 102 (FIG. 1). With additional reference to FIG. 1, each leadless pacemaker 102 is substantially enclosed in a hermetic housing 151 suitable for placement on or attachment to the inside or outside of a cardiac chamber, such as the right atrium and/or right ventricle of the patient heart 152. The hermetic housing 151 defines a sealed cavity 158 (schematically depicted by dashed lines in FIG. 1). The sealed cavity 158 is sized and shaped for containing battery material for powering the pacemaker 102 and pacing, sensing, and communication electronics and control circuitry. Attachment of a leadless pacemaker to the cardiac tissue can be accomplished via a helical anchor 103 on an anchor mount 155 extending from a distal end of the leadless pacemaker. In the description that follows, the pacemaker devices may be placed or implanted within the epicardium 153 (i.e., the outermost layer) of the heart 152.

As can be understood from FIG. 1, the leadless pacemaker 102 can have two or more electrodes 154, 156 located within, on, or near the housing 151, for sensing and/or delivering electrical activity at the muscle of the cardiac chamber and optionally for sensing electrical activity from the muscle, and for bidirectional communication with at least one other device within or outside the body. For example, the housing 151 can have a longitudinal axis A, and the electrode 154 can be a distal pacing electrode mounted on the housing along the longitudinal axis A. The housing 151 can act as the electrode 156 (e.g., a ring electrode). The ring electrode 156 can be the anode and the distal electrode 154 can be the cathode. In some embodiments, the housing 151 can include a conductive material such as titanium, 316L stainless steel, or other similar materials, and is partially coated with an insulating or dielectric (e.g., polymeric) coating, with the uncoated region of the housing 151 defining the electrode 156 that is proximal to the distal electrode 154.

The housing 151 can contain, within the sealed cavity 158, a primary battery to provide power for pacing, sensing, and communication, which may include, for example bidirectional communication. The housing 151 can also contain, within the sealed cavity 158, circuits for sensing cardiac activity from the electrodes 154, 156. The housing 151 may contain circuits for receiving information from at least one other device via the electrodes 154, 156 and contains circuits for generating electrical signals for delivery and/or receiving electrical signals via the electrodes. The housing 151 may contain circuits for transmitting information to at least one other device via the electrodes 154, 156 and can optionally contain circuits for monitoring device health. The housing 151 may contain circuits for controlling these operations in a predetermined manner.

The leadless pacemaker 102 includes a header assembly 110 that can be mounted on a distal end of the housing 151 along the longitudinal axis A. The header assembly 110 can include an electrical feedthrough assembly including an electrical feedthrough (not shown) and the distal electrode 154, e.g., a pacing tip. Example electric feedthrough assemblies are described, for example, in U.S. Pat. No. 11,247,059, issued on February 15, 2022, entitled "Biostimulator having flexible circuit assembly". The header assembly 110 can include the anchor mount 155 mounted on the electrical feedthrough assembly around the longitudinal axis A. A fixation element (e.g., the helical screw 103) is mounted on the anchor mount 155 along the longitudinal axis A. The assembled components of the leadless pacemaker 102 can provide a distal region that attaches to a target tissue, e.g., via engagement of the fixation element with the target tissue. The distal region can deliver a pacing impulse to the target tissue, e.g., via the distal electrode 154 that is held against the target tissue.

As described above, the hermetic housing 151 defines a sealed cavity 158 that can contain electronics and circuitry. These may be disposed in an electronics compartment 160 of the sealed cavity. More particularly, the housing 151 can have a housing wall, e.g., a cylindrical wall, laterally surrounding the electronics compartment 160. In an embodiment, the housing wall has an inner surface extending around the electronics compartment 160 on the longitudinal axis A. The housing wall can include a conductive, biocompatible, inert, and anodically safe material such as titanium, 316L stainless steel, or other similar materials, to laterally enclose the electronics compartment 160. The electronics compartment 160 can be axially enclosed at a proximal end by the battery. More particularly, a distal surface or face of the battery can define the proximal end of the electronics compartment 160. The electronics compartment 160 can be axially enclosed at a distal end by the header assembly 110. More particularly, a proximal surface of the header assembly 110 can define the distal end of the electronics compartment 160. The housing 151 can be attached, e.g., threaded, adhered, or welded, to the header assembly 110 and the battery. Accordingly, the electronics compartment 160 can be contained between the battery, the inner surface of the housing 151, and the header assembly 110.

In an embodiment, as described for example in U.S. Pat. No. 11,247,059, a flexible circuit assembly is contained within the electronics compartment 160. The flexible circuit assembly can include a flexible substrate having one or more electronic components mounted on a flexible substrate. For example, the flexible circuit assembly can include one or more passive electronic components, e.g., capacitors, and one or more active electronic components, e.g., processors. The electronic components can be interconnected by electrical traces, vias, or other electrical connectors. In an embodiment, the electronics assembly includes one or more electrical connectors, e.g., socket and pin connectors or metallized contact pads, to connect to the battery and the electrical feedthrough assembly. For example, the electrical connector can be a socket connector or a metallized pad to receive and/or connect to an electrode pin or a terminal pin.

The electrical connectors of the flexible circuit assembly can be accidentally short-circuited to other conductive components of the leadless pacemaker 102 such as the housing 151 or battery. To reduce the likelihood of such an event, the leadless pacemaker 102 may incorporate components to electrically insulate and/or protect the flexible circuit assembly components from short-circuiting. For example, the leadless pacemaker can include an end insulator (not shown) that includes a planar structure to form a wall between the flexible circuit assembly and the energy source. Suitably, the end insulator can separate the battery, and more particularly an enclosure of the battery, from the flexible circuit assembly. The leadless pacemaker 102 may also include a wall insulator that separates the flexible circuit assembly from the inner surface of the housing 151. It will be appreciated that the flexible substrate of the flexible circuit assembly may provide sufficient insulation and separation from the housing 151 and the battery, and thus, the end insulator and the wall insulator are optional.

The leadless pacemaker 102 can be adapted for delivery and implantation into tissue in the human body. As described above, the leadless pacemaker 102 can be adapted for implantation adjacent to heart tissue on the inside or outside wall of a cardiac chamber, using two or more electrodes located on or within the housing of the leadless cardiac pacemaker for pacing the cardiac chamber upon receiving a triggering signal from at least one other device within the body.

Leadless pacemakers or other leadless biostimulators are typically fixed to an intracardial implant site by an actively engaging mechanism or primary fixation mechanism such as a screw or helical member 103 that screws into the myocardium. Examples of such leadless biostimulators are described in the following publications: (1) U.S. Pat. No. 8,457,742, issued on Jun. 4, 2013, entitled "Leadless Cardiac Pacemaker System For Usage In Combination With An Implantable Cardioverter-Defibrillator"; (2) U.S. Pat. No. 9,358,400 issued on Jun. 7, 2016, entitled "Leadless Cardiac Pacemaker"; (3) U.S. Pat. No. 9,216,298, issued on Dec. 22, 2015, entitled "Leadless Cardiac Pacemaker System with Conductive Communication"; (4) U.S. Pat. No. 8,352,025 issued on Jan. 8, 2013, entitled "Leadless Cardiac Pacemaker Triggered by Conductive Communication"; (5) U.S. Pat. No. 7,937,148 issued on May 3, 2011, entitled "Rate Responsive Leadless Cardiac Pacemaker"; (6) U.S. Pat. No. 7,945,333 issued on May 17, 2011, entitled "Programmer for Biostimulator System"; (7) U.S. Pat. No. 8,010,209, issued on Aug. 30, 2011, entitled "Delivery System for Implantable Biostimulator"; (8) International Application No. PCT/US2006/040564, filed on Oct. 13, 2006, entitled "Leadless Cardiac Pacemaker and System" and published as WO2007043681A2 on Apr. 26, 2007; and (9) U.S. Pat. No. 11,247,059, issued on February 15, 2022, entitled "Biostimulator having flexible circuit assembly."

In addition to the primary fixation mechanism, such as a helix, some leadless biostimulators may further include a secondary fixation mechanism to provide another feature for keeping the leadless biostimulator in place within the body. Secondary fixation mechanisms can be either active (e.g., the secondary fixation mechanism can actively engage tissue, either within or outside the heart), or can be passive (e.g., the secondary fixation mechanism is not attached to tissue but rather prevents the leadless biostimulator from moving around in the body in the case of accidental detachment). Further details on secondary fixation mechanisms can be found in U.S. Pat. No. 8,527,068, issued on Sep. 3, 2013, entitled "Leadless cardiac pacemaker with secondary fixation capability".

Leadless pacemakers or other leadless biostimulators can be delivered to and retrieved from a patient using any suitable delivery and retrieval systems, such as those described in U.S. Pat. No. 10,856,905, issued on December 8, 2020, entitled "Catheter-based system for delivery and retrieval of a leadless pacemaker". In some implementations of delivery systems, a leadless pacemaker is attached or connected to a delivery system and advanced intravenously into the heart. The delivery system can include features to engage the leadless pacemaker to allow fixation of the leadless pacemaker to tissue. For example, in implementations where the leadless pacemaker includes an active engaging mechanism, such as a screw or helical member, the delivery system can include a docking cap or key configured to engage the leadless pacemaker and apply torque to screw the active engaging mechanism into the tissue. As shown in FIG. 1, the leadless pacemaker 102 can have a proximal attachment feature 124 (e.g., a button and stem) configured for engaging the docking cap or key of the delivery system. Examples of attachment features suitable for delivery and retrieval of the leadless pacemaker 102 are described, for example, in U.S. Pat. No. 11,141,597, issued on October 12, 2021, entitled "Leadless pacemaker having attachment feature". In other implementations, the delivery system includes clips designed to match the shape of a feature on the leadless pacemaker and apply torque to screw the active engaging mechanism into the tissue.

With the foregoing description of leadless pacemaker technology set forth, the description will now proceed with examples of pacemaker devices in accordance with the present disclosure. The foregoing discussion is intended to provide information on leadless pacemaker technology to facilitate a better understanding of the various aspects of the present disclosure, and is not intended to limit the scope of the present disclosure in any respect.

### B. Example Pacemaker Devices

In the examples that follow, pacemaker devices are described that include a proximal pacemaker portion and a distal extension with an integrated lead that provides reach between the proximal pacemaker portion and a target cardiac site (*e.g*., an intramyocardial, epicardial, or endocardial site) for sensing and/or pacing. The distal extension includes one or more fixation elements for anchoring the distal extension at the cardiac site and one or more distal electrodes for sensing and/or pacing at the cardiac site. The fixation elements may be passive (i.e., do not operate as electrodes) or active (i.e., operate as electrodes). The proximal pacemaker portion houses the electronics and circuitry for electrical signals delivered to and/or received from the electrodes. Advantageously, the proximal pacemaker portion is compact with a relatively small footprint, which increases the versatility of the proximal pacemaker portion with respect to locations where it can be implanted. For example, the proximal pacemaker portion can be sized and shaped for implanting at a site proximate to the patient's heart (e.g., within the rib cage). This in turn allows for an efficient and easier implantation process of the pacemaker device, and can allow for a shorter lead to be used. Other advantages are described elsewhere herein and/or will become apparent upon reading the following description.

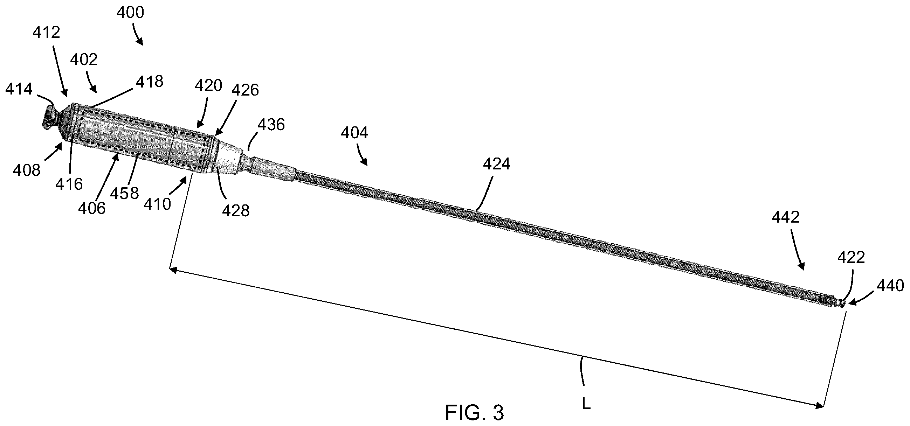

FIG. 3 illustrates a first example of a pacemaker device 400 that includes a proximal pacemaker portion 402 and a distal extension 404. The proximal pacemaker portion 402 can also be referred to as an implantable pulse generator portion or an IPG portion. The proximal pacemaker portion 402 can have a similar configuration as the leadless pacemaker 102 described above, and includes a hermetic housing 406 sized and shaped for placement within a body of a patient in proximity to the heart. Advantageously, the hermetic housing 406 is relatively compact and has a relatively small footprint, which enables the proximal pacemaker portion 402 to be placed within the body of the patient in proximity to the heart. The relatively small size of the proximal pacemaker portion 402 provides a relatively high degree versatility with respect to the locations in the patient within which the proximal pacemaker portion 402 can be placed in proximity to the heart, thereby reducing the usable length necessary for the distal extension 404 described in more detail below. In this regard, the location at which the proximal pacemaker portion 402 is not limited, and can vary depending on various factors including intended application, size of the patient, preferences of the physician, among other considerations. In various applications, the proximal pacemaker portion 402 can be placed behind a patient's sternum or rib cage, in the thorax, in the abdomen, or in another suitable location. In various embodiments, the proximal pacemaker portion 402 has a size or footprint of less than 15 cubic centimeters, such as less than 10 cubic centimeters.

The hermetic housing 406, like the hermetic housing 151, defines a sealed cavity 458 that can contain electronics and a power source (e.g., a battery) for the pacemaker device 400. For example, the housing 406 can contain a primary battery to provide power for pacing, sensing, and communication (e.g., bidirectional communication, circuits for sensing cardiac activity or receiving information from one or more electrodes of the pacemaker device 400, circuits for transmitting information to at least one other device via the electrodes, and, optionally, circuits for monitoring device health, as well as circuits for controlling any of these operations in a predetermined manner. The above description of electronics and circuit assemblies, electrical feedthrough assemblies, and insulators for the leadless pacemaker 102 applies to the pacemaker device 400 unless expressly stated otherwise or the context clearly indicates otherwise.

The hermetic housing 406 extends between a proximal end 408 and a distal end 410 of the proximal pacemaker portion 402. A proximal attachment assembly 412 extends from the proximal end 408 of the housing and is configured to enable delivery and/or retrieval of the pacemaker device 400. The proximal attachment assembly 412 can also be configured for installing the proximal pacemaker portion 402 within the patient. In the illustrated example, the proximal attachment assembly 412 includes a proximal attachment feature 414 (e.g., a button and stem) that is similar to the proximal attachment feature 124 of the leadless pacemaker 102 described above with reference to FIG. 1. In various implementations, the attachment assembly 412 can have any suitable attachment configuration to enable it to function as described herein, including the attachment configurations described in U.S. Pat. No. 11,141,597. The attachment assembly 412 can be attached to the hermetic housing 406 via a flange 416 using any suitable means, such as welding, threads, or an adhesive.

In some embodiments, the housing 406 can act as an electrode 418 for pacing, sensing, and/or communication purposes. Similar to the housing 151 described above for the leadless pacemaker 102, the housing 406 can include a conductive material such as titanium, 316L stainless steel, or other similar materials, and is partially coated with an insulating or dielectric (e.g., polymeric) coating, with the uncoated region of the housing 406 defining the electrode 418 (e.g., a ring electrode). The electrode 418 is illustrated as being proximate or adjacent the proximal end 408 of the proximal pacemaker portion 402, but the electrode 418 may have any suitable location to enable it to function as described.

As can be understood from FIG. 3, the proximal pacemaker portion 402 also includes a header assembly 420 that can be mounted or attached (e.g., welded, threaded, or adhered) to the housing 406 at the distal end 410 of the proximal pacemaker portion 402. The header assembly 420, like the header assembly 110 of the leadless pacemaker 102, can include an electrical feedthrough assembly including an electrical feedthrough (not shown). In the example pacemaker device 400, a distal electrode is not located at the header assembly 420. Instead, a distal electrode 422 is located distal from the header assembly 420 and connected to the electrical feedthrough via a leaded conductor 424, or lead 424, that is part of the distal extension. The distal extension 404, the lead 424, and the distal electrode 422 will be described in more detail below.

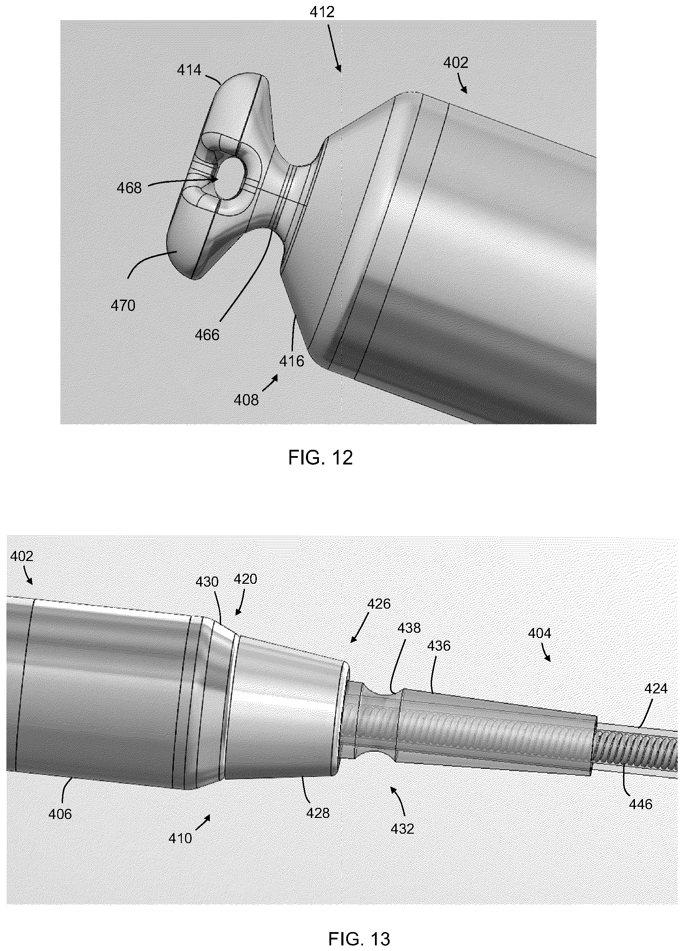

With additional reference to FIG. 13, the distal extension 404 is coupled to the distal end 410 of the proximal pacemaker portion 402 via a coupling assembly 426. In the illustrated example, the coupling assembly 426 includes a coupling collar 428 that is attached (e.g., threaded, welded, or adhered) to a flange 430 of the header assembly 420. The flange 430 of the header assembly 420 is attached (e.g., threaded, welded, or adhered) to the housing 406 at the distal end 410 of the proximal pacemaker portion 402. The coupling collar 428 receives at least a portion of a proximal region 432 of the distal extension 404. In the illustrated example, each of the flange 430 and the coupling collar 428 has a frusto-conical shape, with a tapered outer diameter that reduces in a distal direction. In other examples, the flange 430 and/or the coupling collar 428 may have another suitable shape.

The proximal region 432 of the distal extension 404 includes a proximal end portion of the lead 424 and a strain relief boot 436 slidably mounted around the lead 424 at the proximal end portion thereof. The proximal end region of the lead 424 and at least a portion of the strain relief boot 436 are received by the coupling collar 428, whereby the lead 424 can be electrically connected to the electronics disposed in the housing 406 via the electrical feedthrough assembly of the header assembly 420. The strain relief boot 436 can relieve strain on and stabilize the lead 424 within the coupling collar 428 and relative to the proximal pacemaker portion 402. In some embodiments, the strain relief boot 436 can also serve as a suture sleeve that include an anchoring feature 438 (e.g., a circumferential groove or radially inward projecting recess). The anchoring feature 438 of the strain relief boot 436 can facilitate anchoring the proximal region 432 of the distal extension of the proximal pacemaker portion 402 within the patient (e.g., by tying the suture sleeve to tissue via sutures that wrap around the anchoring feature). Examples of suture sleeves, and examples of cardiac pacing leads including a suture sleeve, are described in U.S. Pat. No. 10,967,175, issued on April 6, 2021, entitled "Cardiac lead with suture sleeve".

As shown in FIG. 3, the distal extension 404 that includes the lead 424 and the distal electrode 422 extends a usable length L, or a length measured when the distal extension 404 is substantially straight relative to a longitudinal axis of the device 400, from the distal end 410 of the proximal pacemaker portion 402 to the distal electrode 422 at a distalmost end 440 of the pacemaker device 400. Because the proximal pacemaker portion 402 has a relatively small footprint, which enables greater versatility in the placement of the pacemaker portion within the body of the patient, the distal extension 404 may have a relative short usable length compared to transvenous leads typically used in pacing applications. For example, the distal extension 404 may have a usable length L less than 60 centimeters (cm), such as less than 50 cm. The usable length L may be any suitable length to enable the pacemaker device 400 to function as described, and may vary depending on the intended application as well as size of the patient.

In the illustrated example, the lead 424 is integrated with the proximal pacemaker portion 402 such that the pacemaker device 400 is a singular device. More specifically, the lead 424 is integrated with the electrical feedthrough assembly of the proximal pacemaker portion 402 to define the singularity of the device 400. The lead 424 is electrically connected to the electronics and circuitry of the proximal pacemaker portion 402 via the electrical feedthrough assembly, such that electrical signals can be administered and sensed by the proximal pacemaker portion 402 via the electrical pathways of the lead 424. The electrical pathways of the lead 424 may be established, for example, by an electrical conductor 446 that extends the length L of the distal extension between the proximal region 432 and the distal region 442.

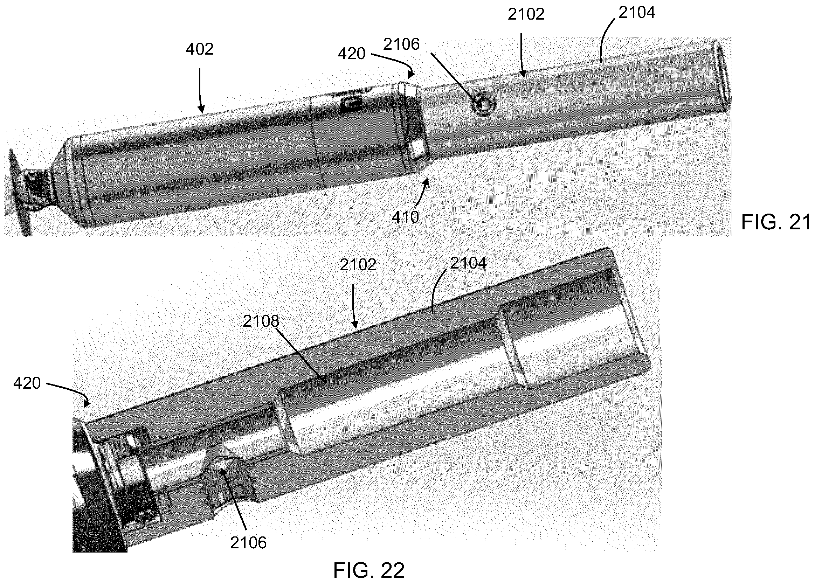

In alternative embodiments, such as those described below with reference to FIGS. 21 and 22, the pacemaker device 400 can be formed by "plugging" the distal extension 404, and more specifically the lead 424, into the header assembly 420 of the proximal pacemaker portion 402 to electrically connect the lead 424 and the electrical feedthrough assembly. In these alternative embodiments, the pacemaker device 400 may be implanted in or attached to any desired location either endocranially, epicardially, or intramyocardially. In such alternative embodiments, the lead 424 can include a connector assembly at the proximal region 432 of the distal extension, which is inserted in the coupling collar 428 and facilitates electrically connected the lead with the electrical feedthrough assembly of the header assembly 420. For example, the connector assembly may be provided with sealing rings and one or more electrical connectors (e.g., ring or pin contacts). The connector assembly can be configured to be plugged into and/or mate with the electrical feedthrough assembly, and the sealing rings can form a fluid-tight seal to prevent the ingress of fluids into the electrical connection region between the lead 424 and the proximal pacemaker portion 402. When the connector assembly is plugged into or mates with the electrical feedthrough assembly, the contacts electrically connect with the electronics and circuitry of the proximal pacemaker portion 402 such that electrical signals can be administered and sensed by the proximal pacemaker portion 402 via the electrical pathways of the lead 424.

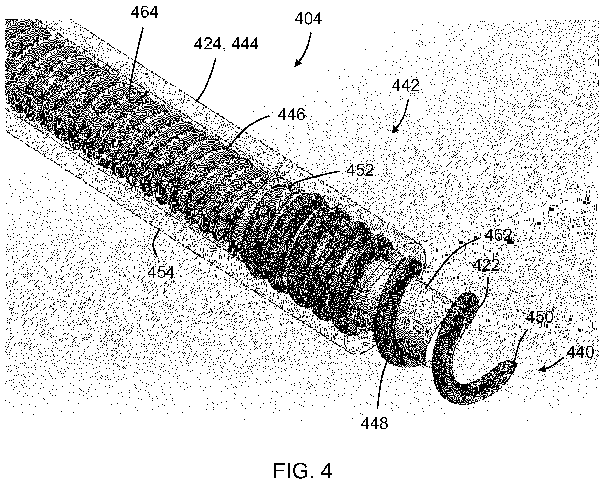

With additional reference to FIG. 4, which depicts a distal region 442 of the distal extension 404 in greater detail, the lead 424 is provided with an elongate lead body 444 that extends between the proximal region 432 and the distal region 442 of the distal extension 404. In the illustrated example, the electrical conductor 446 is a coiled wire. In some embodiments, the electrical conductor 446 may be in the form of helically coiled electrical conductors. In other embodiments, the conductor 446 may be in the form of wires, cables or other electrical conductors that are linear or helically coiled in configuration. The conductor 446 is supported by (e.g., wrapped around) an inner support structure 462 (e.g., a cylindrical rod or plug), as shown in FIG. 4. The inner support structure 462 may be flexible or may be substantially rigid depending on the intended application of the device 400. The inner support structure 462 can be a pharmaceutical (e.g., steroid or drug) element that reduces inflammation at the site of implant. Although the inner support structure 462 is shown in the illustrated embodiment as a cylindrical rod or plug, the support structure 462 can have any suitable form or configuration to enable the structure 462 to function as described.

As shown in FIG. 4, the lead 424 includes a fixation element 448 at the distalmost end 440 of the pacemaker device 400. The fixation element 448 in the form of a helical screw in this example. The helical screw 448 is an active fixation element, meaning that the helical screw 448 operates to anchor the distalmost end 440 of the pacemaker device 400 in cardiac tissue (e.g., intramyocardial tissue, epicardial tissue, or endocardial tissue) and operates as the distal electrode 422 to sense and/or deliver electrical signals. In other examples, such as those described elsewhere herein, a passive fixation element, separate from the distal electrode 422, may additionally or alternatively be included. Passive fixation elements are not configured to operate as electrodes (e.g., do not sense or deliver pacing). In some examples, the distal electrode 422 may not be a fixation element (e.g., may be a dome electrode that contacts tissue but is not anchored within the tissue). It will be appreciated the lead 424, and more broadly the leads of the pacemaker devices in accordance with the present disclosure, encompass implantable leads of all types and of all fixation mechanisms, including passive fixation mechanisms in addition to active fixation mechanisms.

In the illustrated example, the helical screw 448 extends to a screw tip, which defines the distalmost end 440 of the device 400. The screw tip 450 is located distally outward beyond a distal end of the support structure 462 of the lead 424, such that the helical screw 448 can be installed within the cardiac tissue a sufficient depth and, in some instances, reaches the myocardium. The screw tip 450 is contoured (e.g., with a sharp edge) to enable tissue penetration. As will be described in further detail below, the helical screw 448 be driven into the tissue by torque applied to the distal region 442 of the distal extension 404 via an introducer tool. In some examples, during delivery of the pacemaker device 400 to the epicardium, the tip 450 of the helical screw 448 may be retracted proximally from the distalmost end 440, and proximal the distal end of the support structure 462. The helical screw 448 can, in some embodiments, be retracted proximally into the confines of an outer sheath 454 of the lead 424, or by an obturator or other structural member being combined with the helical screw 448, to inhibit the helical screw 448 from being able to penetrate tissue until it is deployed by an operator into the extended state shown in FIG. 4.

The helical screw 448 is situated adjacent to the conductor 446 and mounted on the support structure 462 using a mounting piece 452 (e.g., a mounting ring). The helical screw 448 is in electrical contact with the conductor 446 (e.g., via the mounting piece 452) at the distal end thereof such that electrical signals can be carried to and from the distal electrode 422 via the conductor 446. As described above, in this example, the helical screw 448 is an active fixation element that operates as the distal electrode 422 in addition to providing fixation to cardiac tissue. Where, as in this example, the helical screw 448 is also configured to act as the electrode 422, depending on the dictates of the pacemaker device 400, the helical screw 448 may be employed for sensing electrical energy and/or administration of electrical energy (e.g., pacing). The helical screw 448 is electrically coupled to a contact of the connector assembly of the lead 424 at the proximal region 432, and thereby the electrical feedthrough assembly of the proximal pacemaker portion 402, via the electrical conductor 446.

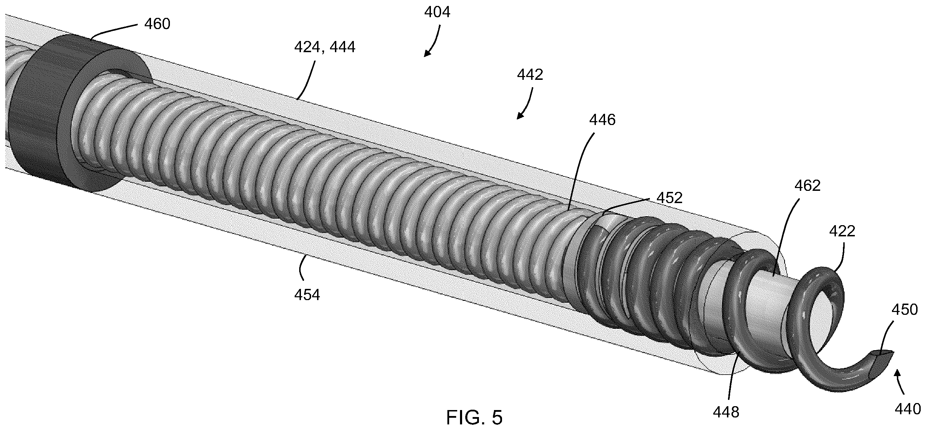

FIG. 5 depicts an alternative embodiment of the distal region 442 of the distal extension 404 in which a bipolar pair of electrodes 422, 460 is used. In this example, the distal region 442 also includes, in addition to the distal electrode 422, an annular ring electrode 460 proximally offset from the distalmost end 440 of the device 400. The annular ring electrode 460 is also spaced an axial distance from and located proximally relative to the distal electrode 422 (i.e., the helical screw 448). Depending on the dictates of the pacemaker device 400, the ring electrode 460 may be employed for sensing electrical energy and/or administration of electrical energy (e.g., pacing). The ring electrode 460 is mounted on and electrically coupled to the conductor 446 such that the ring electrode is electrically coupled to a contact of the connector assembly of the lead 424 at the proximal region 432, and thereby the electrical feedthrough assembly of the proximal pacemaker portion 402, via the electrical conductor 446.

Referring to FIGS. 4 and 5, the lead body 244 includes the outer insulation sheath 454 that defines an inner lumen 464 within which the conductor 446 and the inner support structure 462 are coaxially disposed. In some examples, the outer insulation sheath 454 is fabricated of silicone rubber, polyurethane, silicone rubber-polyurethane-copolymer (SPC), polytetrafluoroethylene (PTFE), or another suitable polymer. The insulation sheath 454 isolates the interior components of the lead 424, including the electrical conductors from each other. Additionally or alternatively, the outer insulation sheath 454 isolates the inner components of the lead 424 from the surrounding environment. The sheath 454 may be single or multi-layer construction.

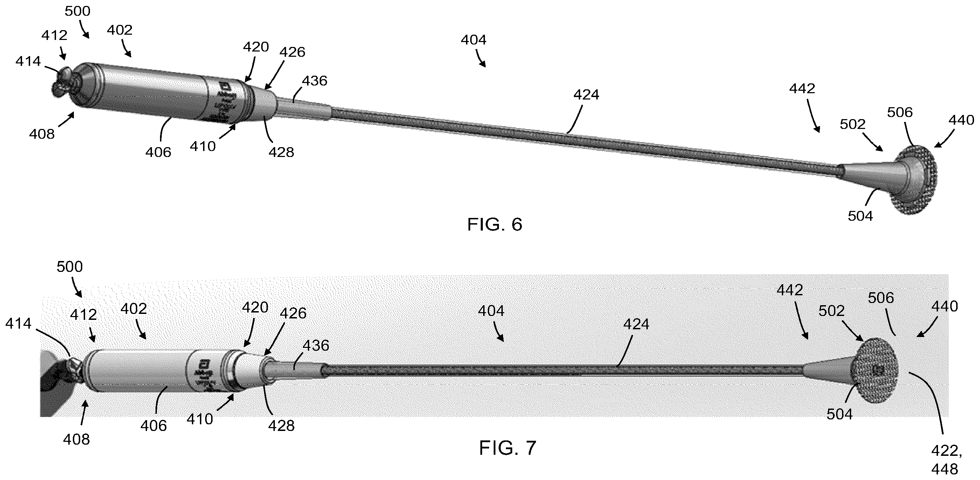

FIGS. 6 and 7 illustrate a second example of a pacemaker device 500 that includes the proximal pacemaker portion 402 and the distal extension 404. The pacemaker device 500 includes the features and components of the pacemaker device 400 shown in FIGS. 3-5, with like reference numerals indicating like features and elements. The above description of the pacemaker device 400 applies to the pacemaker device 500 unless expressly stated otherwise or the context clearly indicates otherwise.

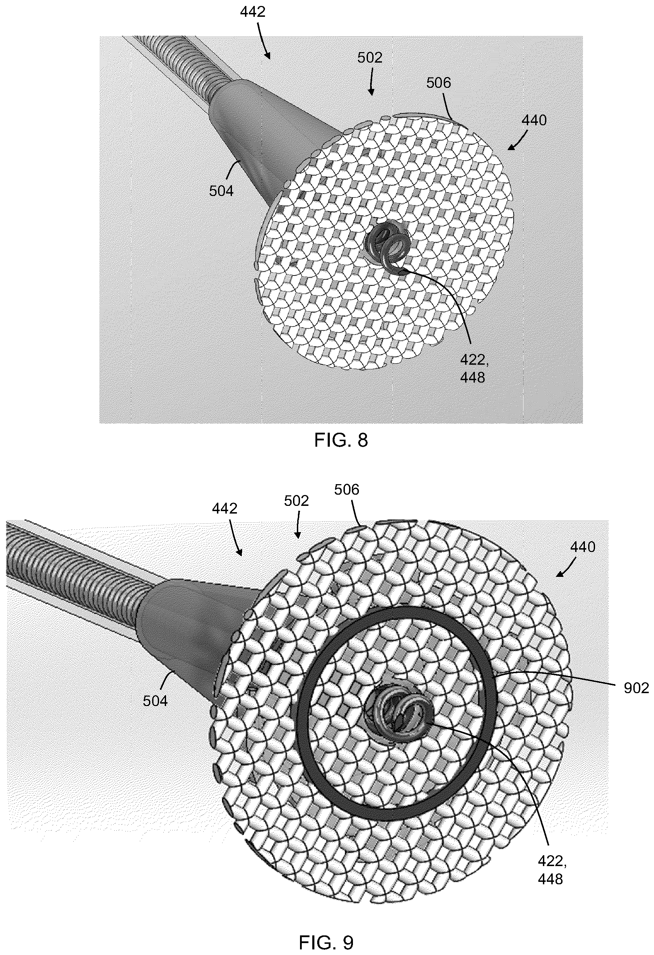

With additional reference to FIG. 8, in this example of a pacemaker device 500, the distal region 442 of the distal extension also includes a passive fixation assembly 502 for implanting the distalmost tip 440 at a cardiac target site, such as an intramyocardial, epicardial, or endocardial target site. The passive fixation assembly 502 is included in addition to the active fixation element 448 (e.g., the helical screw that operates as a unipolar distal electrode 422, shown in FIG. 4, or a bipolar distal electrode 422 with proximal electrode 460, shown in FIG. 5). The passive fixation assembly 502 includes a mounting sleeve 504 slidably mounted around the lead 424 at a distal end portion thereof and a passive fixation element 506 coupled to the lead 424 via the mounting sleeve 504 and extending radially outward from the mounting sleeve 504. The mounting sleeve 504 tapers inwardly in the proximal direction, and has a frusto-conical shape in this example. The mounting sleeve 504 can be shaped to complement a distal end of an introducer or delivery tool for the pacemaker device 500 and can facilitate torque transfer between the tool and the device 500, described in more detail below.

The passive fixation element 506 can be a mesh material made of any suitable biologically compatible material, such as a biologically compatible polymer. For example, the mesh material may include polyester polymer or silicone. The passive fixation element 506 (e.g., mesh) contacts the cardiac surface (e.g., intramyocardial, epicardial, or endocardial surface) to facilitate tissue ingrowth and anchor the distalmost end 440 of the pacemaker device 500 at the target site. In the illustrated example, the passive fixation element 506 is circular in shape, but can include any suitable shape to enable the passive fixation element to function as described.

The fixation elements and electrodes of the pacemaker devices described herein (e.g., the devices 400 and 500) can be used in isolation or in any combination. Thus, one or more electrodes, fixation elements or features thereof described in any embodiment can be used in combination with one or more electrodes, fixation element, or features thereof described in another embodiment. Additionally, in embodiments where two or more fixation elements or two or more distal electrodes are used in combination, one of the fixation elements and one of the distal electrodes can also be used in isolation.

FIGS. 9-11 illustrate alternative examples of the distal region 442 of a pacemaker device (e.g., the pacemaker device 400 and/or 500), in which a passive fixation element 506 and the distal electrode 422 are included in addition to another passive or active fixation element and/or distal electrode. This allows for a smaller distal cathode electrode which may improve electrical function and extend device longevity. Additionally, where an additional active fixation element or distal electrode is included, the additional active fixation element or additional distal electrode can allow for a bipolar distal electrode configuration, with the active fixation element being the anode. Depending on the dictates of the pacemaker device, the additional active fixation element or distal electrode may be employed for sensing electrical energy and/or administration of electrical energy (e.g., pacing). In the examples of FIGS. 9-11, the additional active fixation element or distal electrode is mounted on the passive fixation element 506 and is electrically coupled to the conductor 446 such that the additional active fixation element or distal electrode is electrically coupled to a contact of the connector assembly of the lead 424 at the proximal region 432, and thereby the electrical feedthrough assembly of the proximal pacemaker portion 402, via the electrical conductor 446.

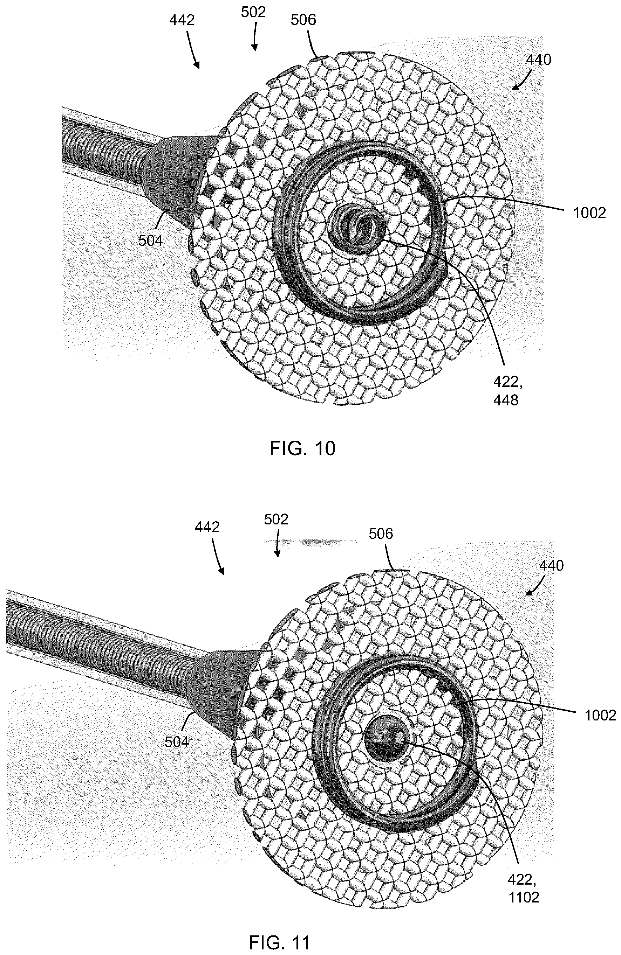

FIG. 9 illustrates an additional distal electrode 902 that is in the form of a ring electrode. The ring electrode 902 can act as an anode in a bipolar configuration with the distal electrode 422 (e.g., the helical screw 448). In this example, the ring electrode 902 is brought into contact with the target site (e.g., an intramyocardial, epicardial, or endocardial site) for sensing and/or pacing, with the helical screw 448 and the passive fixation element 506 serving to anchor the distalmost end 440 to a cardiac tissue, such as the target site tissue. FIG. 10 illustrates an additional distal electrode 1002 that is in the form of a second active fixation element (e.g., a second helical screw). Like the ring electrode 902, the second active fixation element 1002 can act as an anode in a bipolar configuration with the distal electrode 422 (e.g., the helical screw 448). In this example, the second active fixation element 1002 also serves to anchor the distalmost end 440 to the cardiac tissue with the helical screw 448 and the passive fixation element 506. FIG. 11 illustrates an example similar to FIG. 10, except that the helical screw 448 is not included, and is replaced with a domed electrode 1102 that operates as the distal electrode 422 in bipolar configuration with the active fixation element 1002. In this example, the domed electrode 1102 is brought into contact with the target site for sensing and/or pacing, with the active fixation element 1002 (e.g., a helical screw) and the passive fixation element 506 serving to anchor the distalmost end 440 to the cardiac tissue.

As described above, in the pacemaker devices (e.g., the pacemaker devices 400, 500) described herein, the distalmost end 440 is anchored in the target site for sensing and/or pacing and the proximal pacemaker portion 402 can be placed in proximity to the heart, such as behind a patient's sternum or rib cage, in the thorax, in the abdomen, or in another suitable location. The lead 424 provides the reach for transferring electrical signals between the proximal pacemaker portion 402 and the distal electrodes (e.g., 422, 448, 902, 1002, and/or 1102) at the target site. The proximal pacemaker portion 402 is installed at the desired location using anchoring elements (e.g., sutures).

The pacemaker device 400, 500 includes proximal anchoring features for anchoring (e.g., tying) the proximal pacemaker portion 402 at the desired location. Examples of such proximal anchoring features are shown in FIGS. 12 and 13. FIG. 12 is an enlarged view of the proximal end 408 of the proximal pacemaker portion 402, showing the proximal attachment feature 414 (e.g., a button and stem). The proximal attachment feature 414 includes a stem 466 and a button or head 470 that is flared radially outward relative to the stem 466, and a hollow portion 468. The relatively narrow stem 466 and flared button 470 can cooperate to allow non-absorbing suture material to be tied around and held in place on the stem 466 between the flange 416 and the button 470 and/or tied through hollow portion 468. The suture material can then be used to anchor (e.g., tie) the proximal pacemaker portion 402 at the desired location. Additionally or alternatively, as described above with reference to FIG. 13, the suture sleeve 436 can include an anchoring feature 438 (e.g., a circumferential groove or radially inward projecting recess) that facilitates anchoring the proximal region 432 of the distal extension of the proximal pacemaker portion 402 within the patient (e.g., by tying the sleeve 436 to tissue via sutures that wrap around the anchoring feature).

### C. Example Delivery Systems and Methods for Pacemaker Devices

Example delivery tools or introducers suitable for implanting the pacemaker devices of the present disclosure will now be described. As described above, the pacemaker devices (e.g., the pacemaker devices 400, 500) described herein include a proximal pacemaker portion 402 with a relatively small footprint, enabling the device to be suitably sized for pediatric, and more specifically neonatal use, and/or to provide greater versatility with the respect to implant locations of the pacemaker portion, shorter lead length, and more efficient and easier implantation procedures for the device.

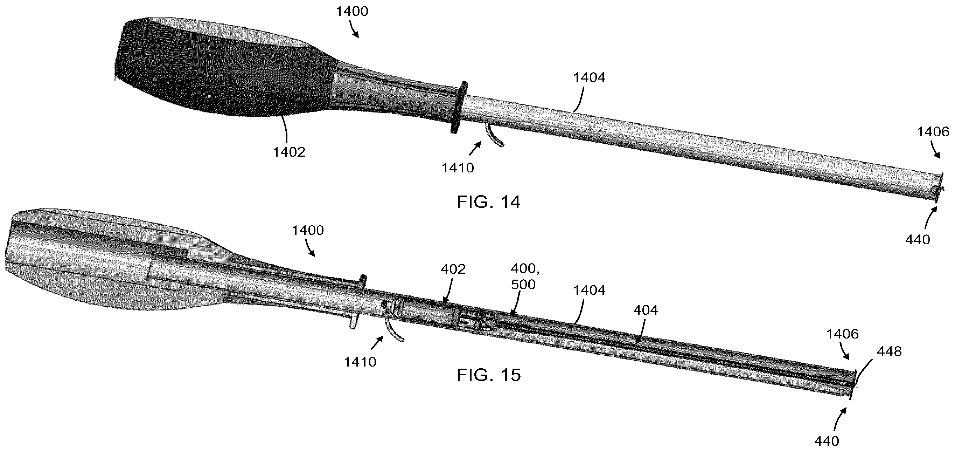

FIGS. 14 and 15 illustrate an example delivery tool 1400 that can be used to implant the pacemaker device 400, 500 at a target site. Advantageously, since the pacemaker device 400, 500 described herein can be entirely implanted in proximity to the heart and can be implanted without using transvenous leads, the delivery tool 1400 can be configured as a compact, relatively simplistic tool that is suitable for a subxiphoid or retrocostal implant approach, or another relatively low-risk implant procedure to implant the pacemaker device 400, 500 underneath or near the rib cage of the patient. The delivery tool 1400 includes a handle 1402 and a delivery tube 1404 attached to a distal end of the handle 1402. The handle 1402 defines a central bore within which a proximal portion of the delivery tube 1404 is positioned, as shown in FIG. 15. The delivery tube 1404 is generally cylindrical and is hollow to receive the pacemaker device 400, 500 therein, as shown in FIG. 15. The delivery tube 1404 extends distally to a distalmost end 1406 of the delivery tool 1400. The delivery tube 1404 can be generally stiff and rigid (e.g., a metal hypotube), since the tool 1400 does not need to take a transvenous approach to deliver the device.

As shown in FIG. 15, when the pacemaker device 400, 500 is loaded in the delivery tube 1404, the proximal pacemaker portion 402 is located proximate the handle 1402 and the distal extension 404 extends to the distalmost end 440 which is adjacent the distalmost end 1406 of the delivery tool 1400. During an implant procedure, the distalmost end 440 of the pacemaker device 400, 500 may extend slightly outward beyond the distalmost end 1406 of the delivery tool 1400, such that the fixation element(s) (e.g., the helical screw 448) of the pacemaker device 400, 500 may be brought into contact with and penetrate the target site of the patient's heart using the delivery tool 1400. In some implementations, the pacemaker device 400, 500 can be moveable axially within the delivery tube 1404 such that the distalmost end 440 can be deployed distally outward beyond the distalmost end 1406 of the tool 1400 and retracted such that the distalmost end 440 is either flush with or proximal to the distalmost end 1406 of the tool 1400.

The delivery tool 1400 cooperates with the pacemaker device 400, 500 to implant (e.g., screw) the distalmost end 440 into the target site tissue. For example, an operator of the tool 1400 can transmit torque to a fixation element (e.g., helical screw 448) by rotating the tool 1400 when the distalmost end 440 is brought into contact with the target site tissue at the desired implant site, to thereby penetrate the tissue with the fixation element and anchor the fixation element in the tissue (e.g., by screwing the fixation element into the tissue). Torque can be transmitted to the pacemaker device 400, 500 by rotating the tool, with the pacemaker device 400, 500 rotatably fixed relative to the delivery tube 1404, such that rotation of the tool 1400 causes rotation of the pacemaker device.

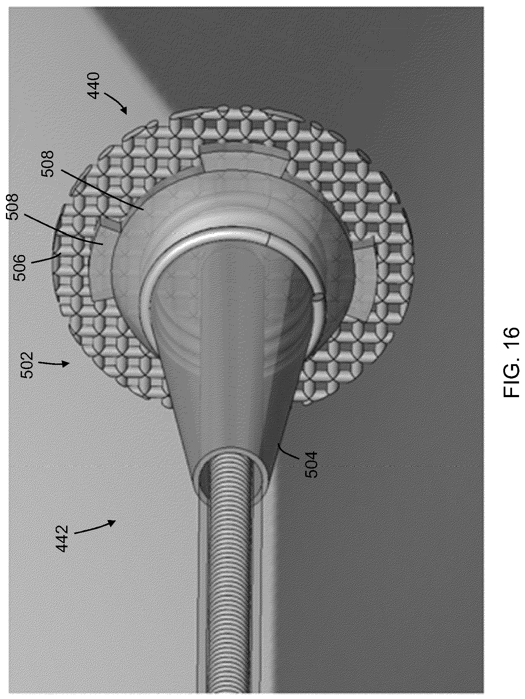

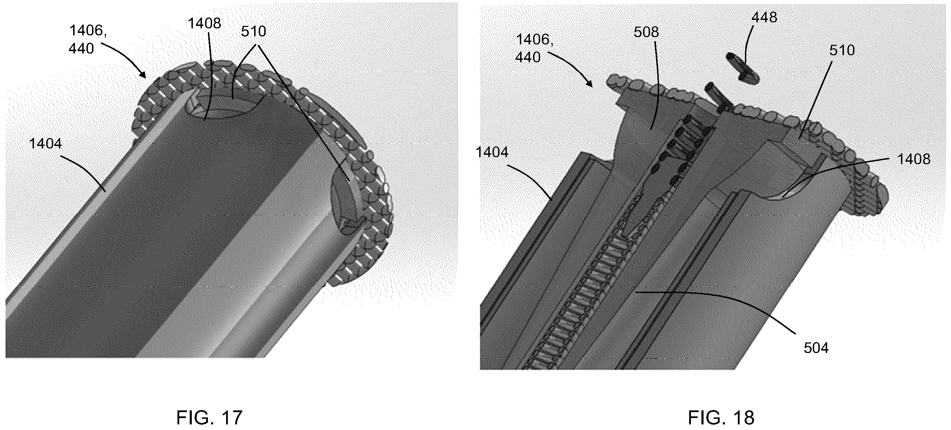

With additional reference to FIGS. 16-18, the distalmost end 1406 of the tool 1400 and the distal region 442 of the distal extension 404 may have complementing features (e.g., keys and keyways, teeth and grooves, or any suitable complementing mechanical features) that rotatably fix the pacemaker device 400, 500 relative to the tube 1404. In the illustrated example, the mounting sleeve 504 of the pacemaker device 400, 500 includes a distal flange 508 and bosses or teeth 510 extending radially outward from the distal flange. The delivery tube 1404 includes a series of slots or grooves 1408 at the distalmost end 1406. As shown in FIGS. 17 and 18, when then pacemaker device 400, 500 is loaded in the tool 1400, each one of the teeth 510 is received by and fits within a respective one of the grooves 1408. The teeth 510 and grooves 1408 interact to rotatably fix the pacemaker device 400, 500 relative to the tube 1404 and thereby enable the operator to transfer torque to the pacemaker device 400, 500 by rotating the tool 1400 and implant the fixation element 448 (e.g., by screwing the helical screw) into the target site tissue.

The delivery tool 1400 also includes a retention and release mechanism 1410 for selectively retaining the pacemaker device 400, 500 within the delivery tube 1404 and releasing the pacemaker device from the tube when the pacemaker device has been implanted at the target site. The mechanism 1410 can interact with the proximal end 408 of the proximal pacemaker portion 402 of the pacemaker device 400, 500, such that the trigger mechanism 1410 prevents axial motion within the delivery tube 1404, and when the trigger is engaged by the operator the mechanism 1410 releases and/or pushes the proximal pacemaker portion 402 of the device 400, 500 out of the delivery tool 1400.

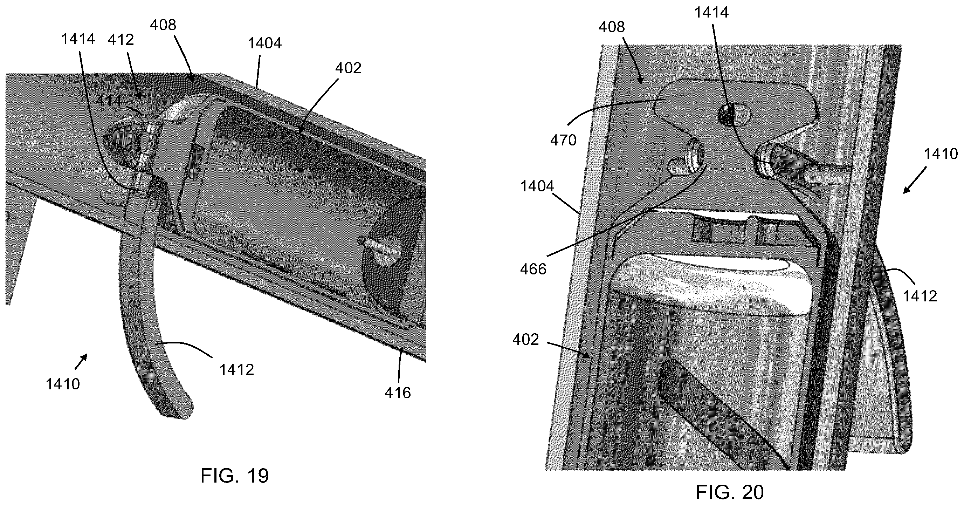

One example of a retention and release mechanism 1410 is shown in FIGS. 19 and 20. In the illustrated example, the retention and release mechanism 1410 includes a finger switch or trigger 1412 that is operably coupled to a clamp 1414 positioned within the delivery tube 1404. The clamp 1414 interacts with the proximal attachment feature 414 of the pacemaker device 400, 500, and is selectively operable using the trigger 1412 between a clamped position and a released position. In particular, the clamp 1414 engages the stem 466 of the attachment feature 412 when in the clamped position to retain the device 400, 500 in the delivery tube 1404, and the clamp 1414 releases the stem 466 when in the released position. Releasing the stem 466 from the clamp 1414 allows axial movement of the device 400, 500 relative to the delivery tube 1404, and enables the device 400 to be released when implanted at the target location.

### D. Examples of Pacemaker Devices With Replaceable Pacemaker Portion

Pacemaker devices, such as those described above, are typically single use systems, designed to be removed and replaced upon depletion of the battery. This may be suitable for patients transitioning to traditional transvenous devices or leadless devices; however, this may not be suitable for patients requiring permanent pacing due to lack of transvenous access. For such patients, it may be desirable to separate the proximal pacemaker portion from the lead for generator change (i.e., device changeout). To facilitate this option, in some examples, the pacemaker portion may have an embedded connector, such as an ISO 5841 IS-1 connector, or it may have a leaded segment and connector. Such connectors are suitably capable of receiving any ISO 5841 IS-1 pacing lead. Such a connector may be configured unipolar or bipolar. In these examples, the pacing lead can be installed using the techniques described herein or using conventional techniques.

FIGS. 21 and 22 illustrate an example of the pacemaker device 400, 500 in which the proximal pacemaker portion 402 includes a connector 2102 (e.g., ISO 5841 IS-1 connector) that allows replacement of the proximal pacemaker portion 402 (e.g., after it has depleted its battery power). The connector 2102 includes a body 2104 (e.g., cylindrical tube) that defines center bore 2108. The bore 2108 receives a lead (e.g., the lead 424 or any conventional pacing lead) and enables electrical connection between the electronics and circuitry housing in the proximal pacemaker portion 402 and the lead. A proximal end of the body 2104 is attached (e.g., threaded, welded, or adhered) to the distal end 410 of the proximal pacemaker portion 402, and more particularly, to the header assembly 420 of the pacemaker portion 402. The connector 2102 also includes a lead lock 2106 that may be accessed via a tool slot in the body 2104 to receive a tool (e.g., screwdriver), and the lead lock 2106 is used to releasably retain the lead in the connector 2102. When the proximal pacemaker portion 402 is ready to be replaced, the lead can be removed from the connector 2102 by releasing the lock 2106, and a new proximal pacemaker portion 402 with a connector 2102 can subsequently be attached to the lead.

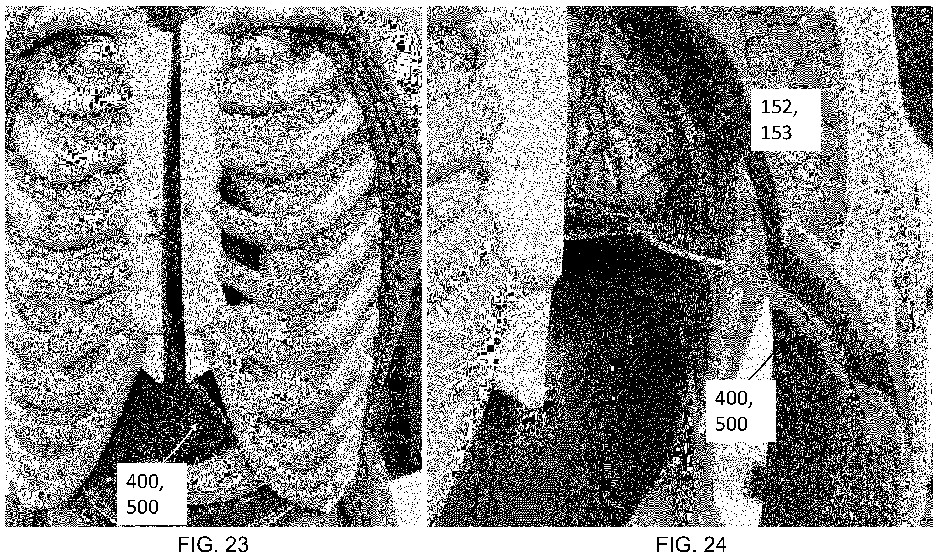

FIGS. 23 and 24 illustrate an example of a pacemaker device 400, 500 according to the present disclosure implanted underneath the rib cage of a patient and implanted within the epicardium 153 of the heart 152. The pacemaker device 400, 500 may be implanted via a subxiphoid, retrocostal, or another implant approach using suitable locations within the rib cage. In particular, FIGS. 23 and 24 illustrate the placement of the pacemaker device 400, 500 via a subxiphoid approach, with the pacemaker portion being secured to the underside of the ribs. This strategic positioning ensures that the pacemaker portion's location relative to the heart 152 remains consistent as the pediatric patient matures and protects the lead from potential conductor damage seen on conventional pacing leads where the lead passes the ribcage in an abdominal implant. The target site of the heart 152 is not limited to any particular location, and the location shown in FIGS. 23 and 24 is by way of example only. In various implementations, the target site may be adjacent an atrium or a ventricle of the heart 152. In some implementations, the target site may be adjacent the right atrium or the right ventricle. The pacemaker portion is kept in proximity to the heart 152 (e.g., installed underneath the rib cage). The versatility of the pacemaker devices of the present disclosure may allow installation at the target sites without using a thoracotomy or other high-risk procedures.

### E. Examples of Pacemaker Device Assemblies

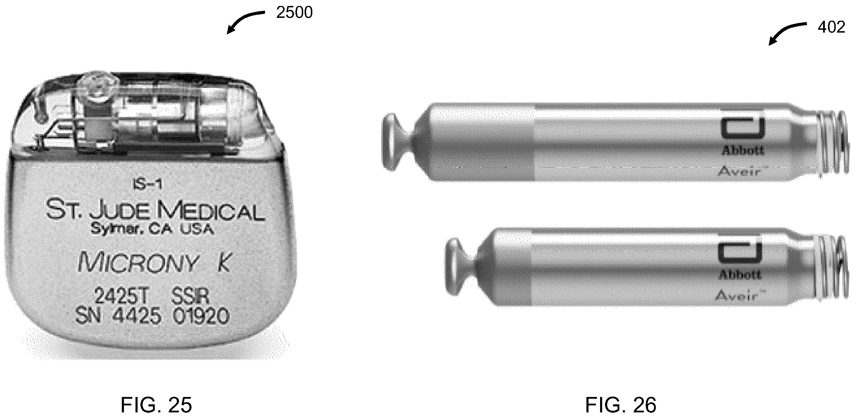

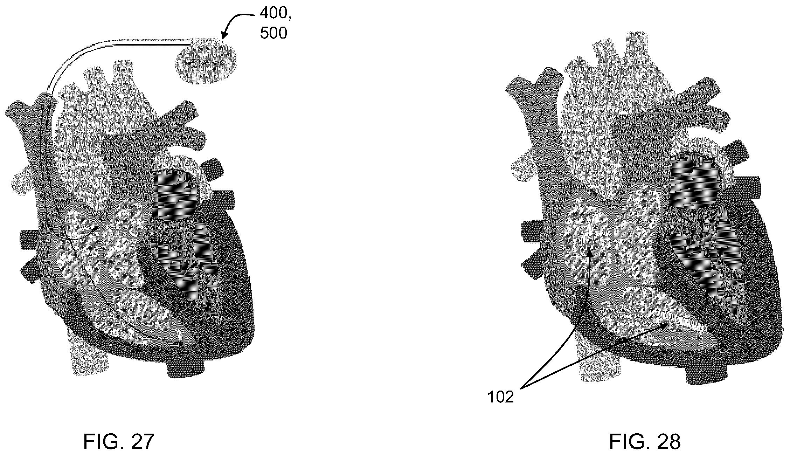

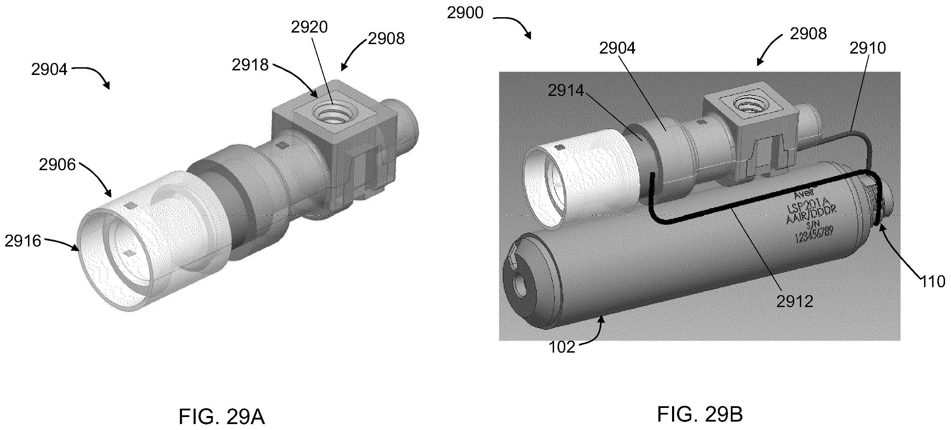

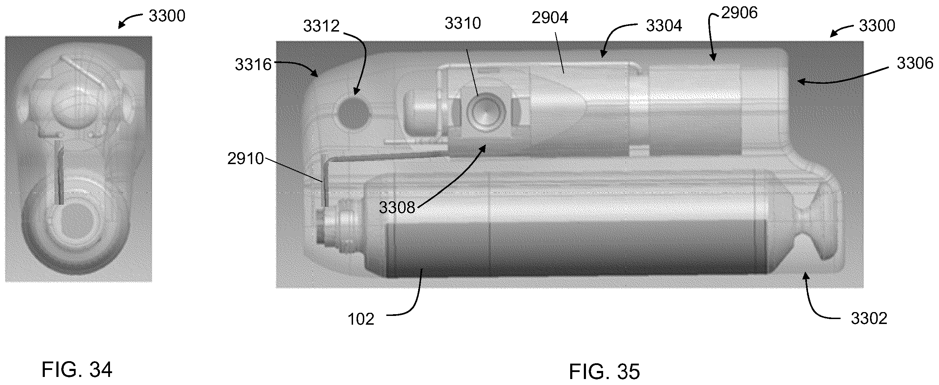

Examples of device assemblies suitable for converting a leadless pacemaker into a leaded pacemaker will now be described. As shown in FIG. 25, example pediatric pacemaker, Abbott Microny^{™}, is no longer in production and no equivalent device is currently available for patients in desire for a small profile (e.g., cosmetic reasons and pediatric patients who are precluded from a larger size pacemaker). As an example, and as shown in FIG. 26, leadless pacemakers, such as Abbott Aveir^{™}, are commercially available and are capable of being converted into a traditional leaded pacemaker, as described in the present disclosure. To facilitate this conversion, in some examples, device assemblies, such as the device assemblies shown in FIGS. 29-36, are provided. With references to FIGS. 27 and 28, examples of placement of leaded pacemaker devices 400, 500 and leadless pacemaker devices 102 are shown. In some examples, the pacemaker devices 102, 400, 500 may be configured in a single chamber configuration which stimulate either a patient's atrium or ventricle. In another example, the pacemaker devices 102, 400, 500 may be configured in a dual chamber configuration to stimulate both the patient's atrium and ventricle by converting two leadless pacemaker devices. In the leaded pacemaker device examples, the pacing lead can be installed using the techniques described herein or using conventional techniques.

FIG. 29A illustrates an example of a connector 2904. The connector 2904 may be similar to the connector 2102 shown in FIG. 21 or may be any other suitable ISO-5841 IS-1 connector. The connector 2904 includes an opening 2916 that enables the connector 2904 to receive a lead (e.g., the lead 424 shown in FIG. 3 or any conventional pacing lead) at a proximal end 2906 of the connector 2904. The connector 2904 also includes an opening 2918 at a distal end 2908. The opening 2918 provides access to a lead lock 2920 of the connector 2904 via a tool slot (not shown) of the lead lock 2920 to receive a tool (e.g., screwdriver). The lead lock 2920 is used to releasably retain the lead in the connector 2904 through the proximal end 2906 of the connector 2904. Other methods of retaining the lead may be used as known by those with ordinary skill in the art.

FIG 29B illustrates a device assembly 2900 that includes a pacemaker device 102 and the connector 2904. The connector 2904 is electrically connected to the pacemaker device 102 through electrical connections 2910 and 2912 (e.g., electrical traces, vias, or other electrical connections). The electrical connection 2910 may be a cathode connection that connects a header assembly 110 (e.g., an electrode, a helical screw, tines, or other mechanisms positioned in the header assembly 110) of the pacemaker device 102 to the distal end 2908 of the connector 2904. The electrical connection 2912 may be an anode connection that connects the header assembly 110 of the pacemaker device 102 to a ring electrode 2914 of the connector 2904. The ring electrode 2914 may act as the anode when the device assembly 2900 is in a bipolar configuration. The electrical connections 2910 and 2912 enable signals to travel, via the connector 2904, between the pacemaker device 102 and the lead 424 (shown in FIG. 3) connected to the proximal end 2906 of the connector 2904. As shown in FIG. 29B, the electrical connections 2910, 2912 are configured in a bipolar manner.

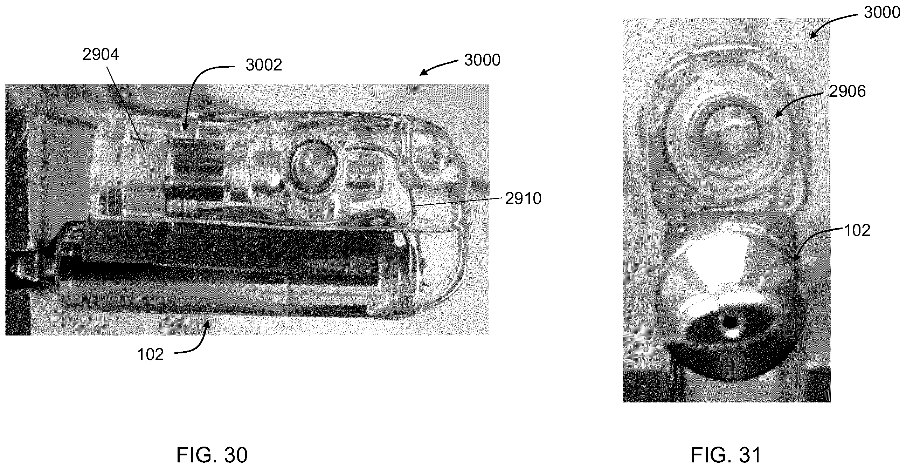

FIGS. 30 and 31 illustrate an example of a device assembly 3000 that includes the pacemaker device 102, the connector 2904, and a housing assembly 3002 enclosing the connector 2904 and partially enclosing the pacemaker device 102. In some examples, the housing assembly 3002 may partially enclose the connector 2904. The housing assembly 3002 may be of a material such as a resin, epoxy, and/or other similar materials. In the present example, the connector 2904 is electrically connected to the pacemaker device 102 through the electrical connection 2910 (e.g., electrical traces, vias, or other electrical connections), which is the cathode connection. As shown in this example, when the pacemaker device 102 is partially enclosed, the device assembly 2900 is in a unipolar configuration with the pacemaker device 102 acting as the anode.

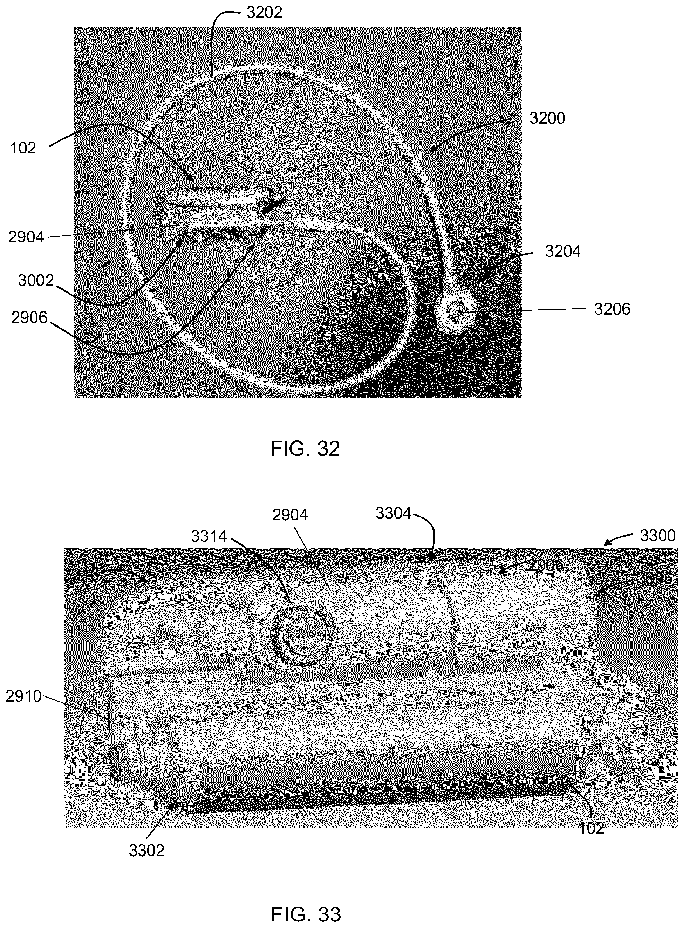

FIG. 32 is a side view of a device assembly 3200 including the pacemaker 102, the connector 2904, and the housing assembly 3002 coupled to a lead 3202 (e.g., the lead 424 shown in FIG. 3 or any conventional pacing lead). The connector 2904 may receive the lead 3202 at the proximal end 2906 of the connector 2904. The connector 2904 enables electric connections between the pacemaker device 102 (e.g., the electronics and circuitry of the pacemaker device 102) and the lead 3202.

In the present example, the lead 3202 may be integrated with the proximal end 2906 of the connector 2904 such that the pacemaker device 102, the connector 2904, and the lead 3202 may form the device assembly 3200. More specifically, the lead 3202 is integrated with the electrical feedthrough assembly of the connector 2904 at the proximal end 2906 to define the singularity of the device assembly 3200. The lead 3202 is electrically connected to the electronics and circuitry of the pacemaker device 102 via the connector 2904 and an electrical feedthrough assembly (e.g. electrical connection 2910 shown in FIG. 29B), such that electrical signals may be administered and sensed by the pacemaker device 102 via the electrical pathways of the lead 3202. The electrical pathways of the lead 3202 may be established, for example, by an electrical conductor (e.g., the electrical conductor 446 shown in FIG. 4) that extends a length between the proximal end 2906 and a distal region 3204 of the lead 3202. The distal region 3204 may include a fixation assembly (such as any fixation assembly described elsewhere herein) for implanting a distalmost tip 3206 at a cardiac target site, such as an intramyocardial, epicardial, or endocardial target site.

FIGS. 33-35 illustrate a respective perspective view, front view, and side view of an example of a device assembly 3300 including the connector 2904, the pacemaker device 102, and a housing assembly 3316 enclosing the connector 2904 and partially enclosing the pacemaker device 102. The housing assembly 3316 includes a first housing 3302 and a second housing 3304. The first housing 3302 is configured to partially enclose the pacemaker device 102. The second housing 3304 is configured to enclose the connector 2904. The second housing 3304 includes an opening 3306 that enables the second housing 3304 to receive, a lead, such as the lead 3202 (shown in FIG. 32) or any conventional pacing lead, at the proximal end 2906 of the connector 2904. The second housing 3304 also includes an opening 3308 that provides access to a lead lock 3310 of the connector 2904. The lead lock 3310 may be accessed via a tool slot (not shown) of the lead lock 3310 that is configured to receive a tool (e.g., screwdriver). The lead lock 3310 is used to releasably retain the lead in the connector 2904 through the proximal end 2906 of the connector 2904. As shown in FIG. 33, opening 3308 is sealed with a septum 3314. The septum 3314 may include a set screw (not shown) and is similarly operable to releasably retain the lead in the connector 2904 through the proximal end 2906 of the connector 2904 via a tool (e.g., hex key). Other methods of retaining the lead may be used as known by those with ordinary skill in the art. In addition, the connector 2904 is electrically connected to the pacemaker 102 through the electrical connection 2910, such as electrical traces, vias, or other electrical connections. In the present example, the pacemaker device 102 and the connector 2904 are connected in a unipolar configuration.

In addition, the housing assembly 3316 includes a hollow portion 3312 that may be used to suture the housing assembly 3320 within a patient's body. The housing assembly 3320 may be placed within the patient's body using delivery tools or directly by hand.

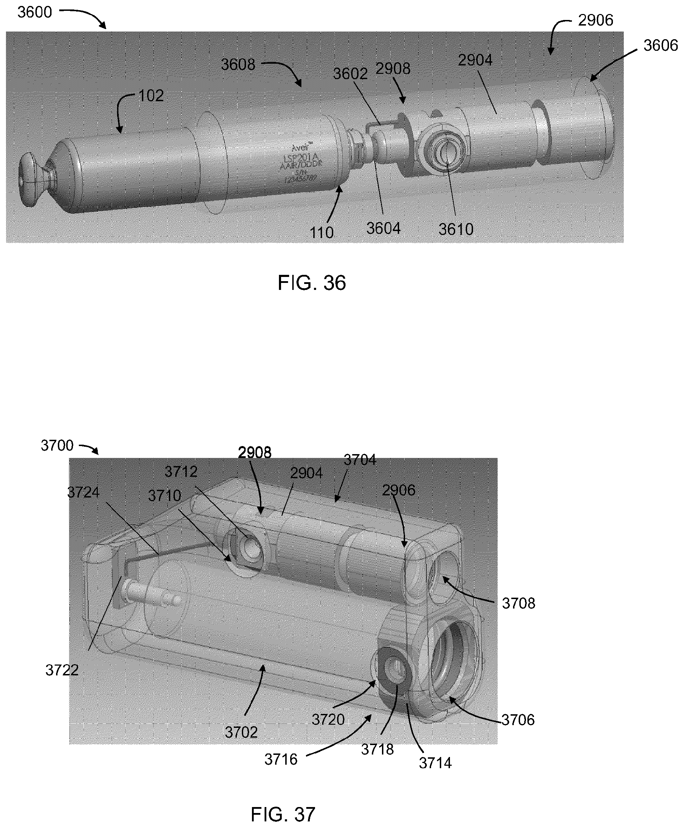

FIG. 36 depicts an alternative example of a device assembly 3600 that includes the pacemaker device 102, the connector 2904, and a housing assembly 3608 in an in-line configuration. The housing assembly 3608 fully encloses the connector 2904 and partially encloses the pacemaker device 102. The pacemaker device 102 is electrically connected to the connector 2904 through electrical connections 3602 (similar to electrical connection 2910 shown in FIG. 29B), such as electrical traces, vias, or other electrical connections. In this alternative example, the distal end 2908 of the connector 2904 is in direct contact with a header assembly 110 via an electrode 3604 of the header assembly 110 of the pacemaker device 102. It should be understood that the connector 2904 may be in contact with the header assembly 110 via a helical screw, tines, or other mechanisms positioned in the header assembly 110 of the pacemaker device 102. In this example, the connection between the pacemaker device 102 and the connector 2904 is shown as a unipolar configuration.

In addition, the housing assembly 3608 includes an opening 3606 that enables the housing assembly 3608 to receive a lead (e.g., the lead 3202 shown in FIG. 32 or any conventional pacing lead) at the proximal end 2906 of the connector 2904. The housing assembly 3608 also includes an opening (not shown) that provides access to a lead lock (not shown) of the connector 2904. The lead lock may be accessed via a tool slot (not shown) of the lead lock that is configured to receive a tool (e.g., screwdriver) . The lead lock is used to releasably retain the lead in the connector 2904 through the proximal end 2906 of the connector 2904. In this example, septum 3610 (similar to septum 3314 shown in FIG. 33) may include a set screw (not shown) and is similarly operable to releasably retain the lead in the connector 2904 through the proximal end 2906 of the connector 2904 via a tool (e.g., hex key). Other methods of retaining the lead may be used as known by those with ordinary skill in the art.

### F. Examples of Adaptors for Pacemaker Devices

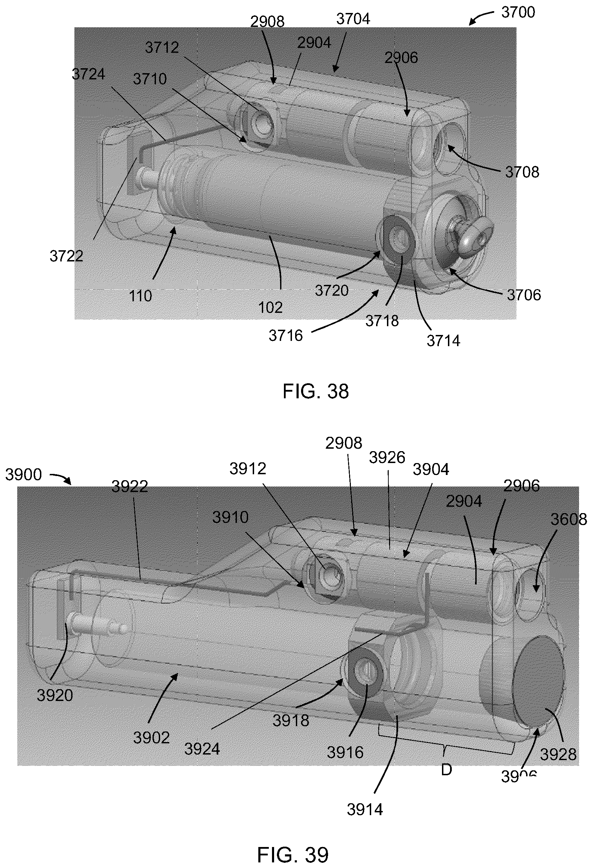

FIGS. 37 and 38 illustrate perspective views of an example of an unipolar adaptor 3700 configured to receive the pacemaker device 102 and the connector 2904. The unipolar adaptor 3700 may be made of a material such as a resin, epoxy, and/or other similar materials. The unipolar adaptor 3700 includes a first housing 3702 and a second housing 3704. The first housing 3702 is configured to receive the pacemaker device 102 through an opening 3706 of the first housing 3702. The second housing 3704 includes an opening 3708 that enables the second housing 3704 to receive the connector 2904. The opening 3708 also enables the connector 2904 to receive a lead (e.g., the lead 3202 shown in FIG. 32 or any conventional pacing lead) at the proximal end 2906 of the connector 2904. The second housing 3704 also includes an opening 3710 that provides access to a lead lock 3712 of the connector 2904 via a tool slot (not shown) of the lead lock 3712 to receive a tool (e.g., screwdriver). The lead lock 3712 is used to releasably retain the lead in the connector 2904. The lead lock 3712 may be sealed by a septum 3314 (as shown in FIG. 33). Other methods of retaining the lead may be used as known by those with ordinary skill in the art.

In this implementation, the first housing 3702 also includes an isolation seal 3714 (e.g., isolation seals found on DF4/IS4 device headers) that is configured to create electrical isolation between the anode and the cathode contacts of the pacemaker device 102 and the connector 2904. The isolation seal 3714 may be configured to create electrical isolation between the anode contacts of the pacemaker device 102 and the exterior of the unipolar adaptor 3700. In this example, the isolation seal 3714 is integrated with the anode contacts of the pacemaker device 102 and is positioned at a proximal end 3716 of the first housing 3702. In other examples, the isolation seal 3714 may be separate from the anode contacts. In some examples, the isolation seal 3714 may be positioned at another location of the first housing 3702.

In addition, the isolation seal 3714 includes a retention set bore 3718 operable to receive a set screw to releasably retain the pacemaker device 102 in the first housing 3702. The set screw may access the retention set bore 3718 via an opening 3720 of the first housing 3702. Other methods of retaining the pacemaker device 102 may be used as known by those with ordinary skill in the art.

In this example, the pacemaker device 102 is electrically connected to the connector 2904 via a contact 3722 of the first housing 3702 and an electrical connection 3724 (similar to the electrical connection 2910 shown in FIG. 29B), such as electrical traces, vias, or other electrical connections. The contact 3722 is configured to connect with a header assembly 110 (e.g., an electrode, a helical screw, tines, or other mechanisms positioned in the header assembly 110) of the pacemaker device 102, and to connect with one end of the electrical connection 3724. The other end of the electrical connection 3724 is connected to a distal end 2908 of the connector 2904. In this implementation, the connection between the pacemaker device 102 and the connector 2904 is unipolar.

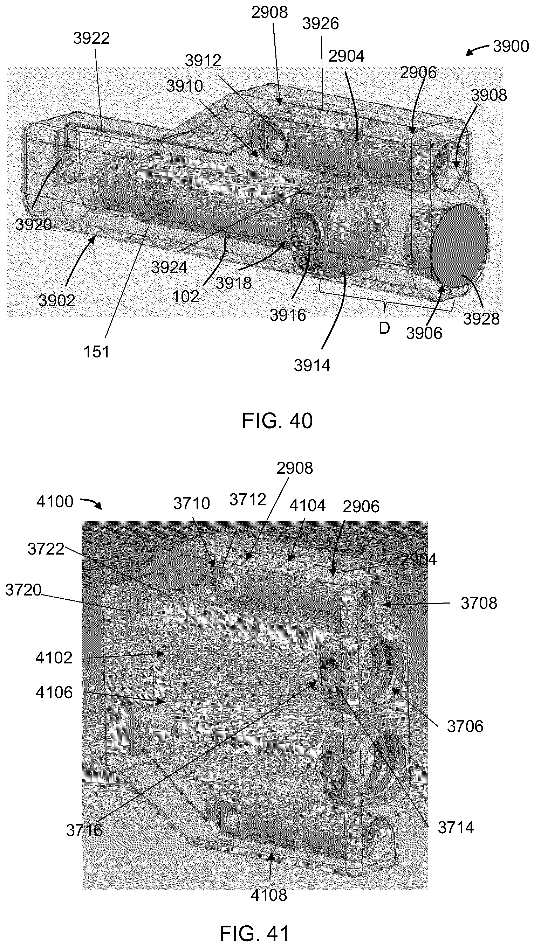

FIGS. 39 and 40 illustrate perspective views of an example of a bipolar adaptor 3900 configured to receive the pacemaker device 102 and the connector 2904. The adaptor 3900 may be made of a material such as a resin, epoxy, and/or other similar materials. The bipolar adaptor 3900 includes a first housing 3902 and a second housing 3904. The first housing 3902 is configured to receive the pacemaker device 102 through an opening 3906 of the first housing 3902. The second housing 3904 includes an opening 3908 that enables the second housing 3904 to receive the connector 2904. The opening 3908 also enables the connector 2904 to receive a lead (e.g., the lead 3202 shown in FIG. 32 or any conventional pacing lead) at the proximal end 2906 of the connector 2904. The second housing 3904 also includes an opening 3910 that provides access to a lead lock 3912 of the connector 2904 via a tool slot (not shown) of the lead lock 3912 to receive a tool (e.g., screwdriver). The lead lock 3912 is used to releasably retain the lead in the connector 2904. The lead lock 3912 may be sealed by a septum 3314 (as shown in FIG. 33). Other methods of retaining the lead may be used as known by those with ordinary skill in the art.

In this implementation, the first housing 3902 includes an isolation seal 3914 (e.g., isolation seals found on DF4/IS4 device headers) that is configured to create electrical isolation between the anode and the cathode contacts of the pacemaker device 102 and the connector 2904. The isolation seal 3914 may be configured to create electrical isolation between the anode and the exterior of the bipolar adaptor 3900. In this example, the isolation seal 3914 is integrated with the anode contacts of the pacemaker device 102 and is positioned at a distance D from the opening 3906 of the first housing 3902 to the isolation seal 3914. In other examples, the isolation seal 3914 may be separate from the anode contacts. In some examples, the isolation seal 3714 may be positioned at another location of the first housing 3902. In addition, the isolation seal 3914 includes a retention set bore 3916 operable to receive a set screw to releasably retain the pacemaker device 102 in the first housing 3702. The set screw may access the retention set bore 3916 via an opening 3918 of the first housing 3702. Other methods of retaining the pacemaker device 102 may be used as known by those with ordinary skill in the art.

In this example, the pacemaker device 102 is electrically connected to the connector 2904 via a contact 3920 of the first housing 3902 and an electrical connection 3922 similar to the electrical connection 2910 shown in FIG. 29B. The pacemaker device 102 is also electrically connected to the connector 2904 via the isolation seal 3914 and an electrical connection 3924 similar to the electrical connection 2912 shown in FIG. 29B. Electrical connections 3922 and 3924 may include electrical traces, vias, or other electrical connectors. The contact 3920 is configured to connect with the header assembly 110 (e.g., an electrode, a helical screw, tines, or other mechanisms positioned in the header assembly 110) of the pacemaker device 102, and to connect with one end of the electrical connection 3922. The other end of the electrical connection 3922 is connected to the distal end 2908 of the connector 2904.

In this example, the isolation seal 3914 is configured to connect with the housing 151 (e.g., an electrode positioned in the housing 151) of the pacemaker device 102, and to connect with one end of the electrical connection 3924. The other end of the electrical connection 3924 is connected to a body 3926 (similar to the body 2104 shown in FIG. 21) of the connector 2904. In examples where the isolation seal 3914 is separate from the anode contacts of the pacemaker device 102, the electrical connection 3924 may be connected directly to the anode contacts of the pacemaker device 102 instead of the isolation seal 3914.

In this implementation, the connection between the pacemaker device 102 and the connector 2904 is bipolar, where the anode contacts of the pacemaker device 102 are electrically connected to the anode contacts of the connector 2904. In addition, the isolation seal 3914, a cap 3928 of the bipolar adaptor 3900, and the distance D enable the bipolar configuration. In particular, after the pacemaker device 102 is received into the first housing 3902, the cap 3928 is configured to seal the first housing 3902 via the opening 3906. Furthermore, the distance D enables separation of the pacemaker device 102 from the cap 3928, thereby enhancing isolation of the pacemaker device 102 from the exterior of the bipolar adaptor 3900. As described above, the isolation seal 3914 is configured to create electrical isolation between the anode and the cathode contacts of the pacemaker device 102 and the connector 2904, and to create electrical isolation between the anode contacts of the pacemaker device 102 and the exterior of the bipolar adaptor 3900.

FIGS. 41-43 illustrate perspective views of examples of a dual chamber adaptor configured to receive two pacemaker devices 102 and two connectors 2904. FIG. 41 illustrates a unipolar adaptor 4100 that includes a first housing 4102, a second housing 4104, a third housing 4106, and a fourth housing 4108. The unipolar adaptor 4100 may be made of similar material(s) to the one(s) of the unipolar adaptor 3700 (shown in FIGS. 37 and 38). The first housing 4102 and the third housing 4106, each includes features and components similar to those in the first housing 3702 shown in FIGS. 37 and 38 with like reference numbers indicating like features and elements. The above description of the first housing 3702 applies to the first housing 4102 and the third housing 4106 unless expressly stated otherwise or the context clearly indicates otherwise.

In addition, the second housing 4104 and the fourth housing 4108, each includes features and components similar to those in the second housing 3704 shown in FIGS. 37 and 38 with like reference numbers indicating like features and elements. The above description of the second housing 3704 applies to the second housing 4104 and the fourth housing 4108 unless expressly stated otherwise or the context clearly indicates otherwise. In this implementation, the first housing 4102 is in contact with the second housing 4104 and the third housing 4106; the second housing 4104 is only in contact with the first housing 4102; the third housing 4106 is in contact with the first housing 4102 and the fourth housing 4108; and the fourth housing 4108 is only in contact with the third housing 4106. In other implementations, the housings in the unipolar adaptor 4100 may be positioned in a different manner.

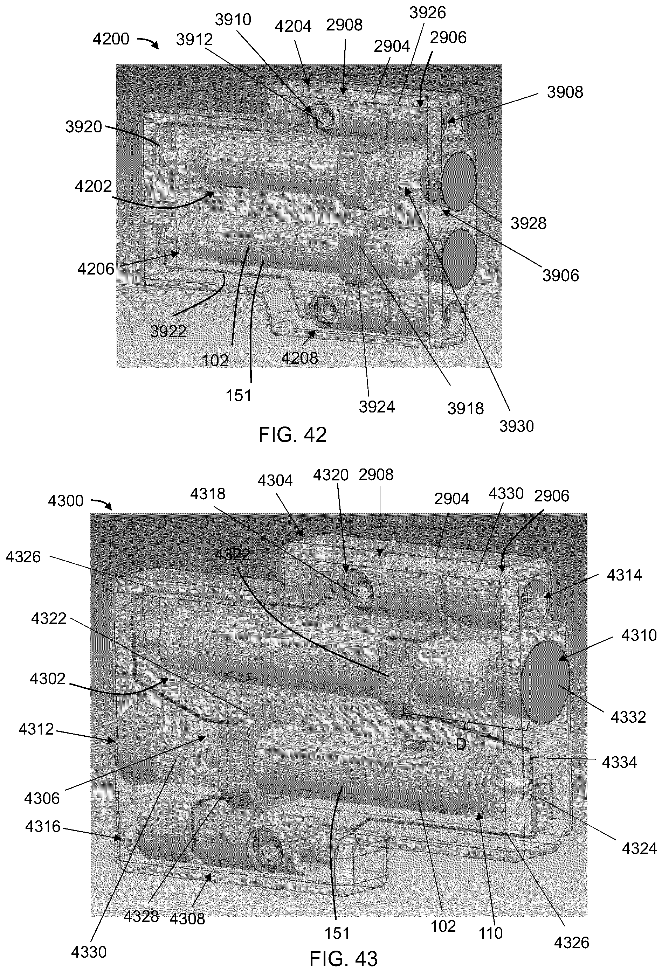

FIG. 42 illustrates a bipolar adaptor 4200 that includes a first housing 4202, a second housing 4204, a third housing 4206, and a fourth housing 4208. The adaptor 4200 may be made of similar material(s) to the one(s) of bipolar adaptor 3900 (shown in FIGS. 39 and 40). The first housing 4202 and the third housing 4206, each includes features and components similar to those in the first housing 3902 shown in FIGS. 39 and 40 with like reference numbers indicating like features and elements. The above description of the first housing 3902 applies to the first housing 4202 and the third housing 4206 unless expressly stated otherwise or the context clearly indicates otherwise.

In addition, the second housing 4204 and the fourth housing 4208, each includes features and components similar to those in the second housing 3904 shown in FIGS. 39 and 40 with like reference numbers indicating like features and elements. The above description of the second housing 3904 applies to the second housing 4204 and the fourth housing 4208 unless expressly stated otherwise or the context clearly indicates otherwise. In this implementation, the first housing 4202 is in contact with the second housing 4204 and the third housing 4206; the second housing 4204 is only in contact with the first housing 4202; the third housing 4206 is in contact with the first housing 4202 and the fourth housing 4208; and the fourth housing is only in contact with the third housing 4206. In other implementations, the housings in adaptor 4200 may be positioned in a different manner.

FIG. 43 illustrates a bipolar adaptor 4300 that includes a first housing 4302, a second housing 4304, a third housing 4306, and a fourth housing 4308. The bipolar adaptor 4300 may be made of a material such as a resin, epoxy, or other similar materials. In this example, the first and second housings 4302, 4304 are displaced on the opposite side of third and fourth housings 4306, 4308.

In this implementation, the first housing 4302 and the third housing 4306 are each configured to receive the pacemaker device 102 via openings 4310 and 4312, respectively. The second housing 4304 and fourth housing 4308 are each configured to receive the connector 2904 via openings 4314 and 4316, respectively. The openings 4314 and 4316 enable second housing 4304 and fourth housing 4308 to each receive a lead (e.g., the lead 3202 shown in FIG. 32 or any conventional pacing lead) at the proximal end 2906 of the connector 2904. The second housing 4304 and fourth housing 4308, each includes an opening 4318 that provides access to a lead lock 4320 of the connector 2904 via a tool slot (not shown) of the lead lock 4320 to receive a tool (e.g., screwdriver). The lead lock 4320 is used to releasably retain the lead in the connector 2904. In other examples, the lead lock 4320 may be sealed by a septum 3314 (as shown in FIG. 33). Other methods of retaining the lead may be used as known by those with ordinary skill in the art.

In this example, the first housing 4302 and third housing 4306, each includes an isolation seal 4322 (e.g., isolation seals found on DF4/IS4 device headers) that is configured to create electrical isolation between the anode and the cathode contacts of the pacemaker device 102 and the connector 2904. The isolation seal 4322 may be integrated with the anode contacts of the pacemaker device 102 and is further configured to create electrical isolation between the anode and the exterior of the bipolar adaptor 4300. In this example, each isolation seal 4322 is integrated with the anode contacts of each pacemaker device 102 and is positioned at a distance D from (a) the opening 4310 of the first housing 4302 to the isolation seal 4322, and (b) the opening 4312 of the third housing 4306 to the isolation seal 4322. In other examples, the isolation seal 4322 may be separate from the anode contacts of the pacemaker device 102. In some examples, the isolation seal 4322 may be positioned at another location of the first housing 4302 and/or third housing 4306.

In addition, each isolation seal 4322 may include a retention set bore (similar to retention set bore 3916 shown in FIG. 39) operable to receive a set screw to releasably retain the pacemaker device 102 in the first housing 4302 and third housing 4306. The set screw may access the retention set bore via an opening (similar to opening 3918 shown in FIG. 39) of each first housing 4302 and third housing 4306. Other methods of retaining the pacemaker device 102 may be used as known by those with ordinary skill in the art.

In this implementation, the first housing 4302 and third housing 4306, each includes a contact 4324. Each pacemaker device 102 is electrically connected to each connector 2904 through each contact 4324 and each electrical connection 4326 (similar to electrical connection 2910 shown in FIG. 29B). Each pacemaker device 102 is also electrically connected to each connector 2904 via each isolation seal 4322 and each electrical connection 4328 (similar to electrical connection 2912 shown in FIG. 29B). Electrical connections 4326 and 4328 may be electrical traces, vias, or other electrical connections. Each contact 4324 is configured to connect with the header assembly 110 (e.g., an electrode, a helical screw, tines, or other mechanisms positioned in the header assembly 110) of each pacemaker device 102, and to connect with one end of each electrical connection 4326. The other end of each electrical connection 4326 is connected to a distal end 2908 of each connector 2904.

In this example, each isolation seal 4322 is configured to connect with the housing 151 (e.g., an electrode positioned in the housing 151) of each pacemaker device 102, and to connect with one end of each electrical connection 4328. The other end of each electrical connection 4328 is connected to a body 4330 (similar to the body 2104 shown in FIG. 21) of each connector 2904. In examples where the isolation seal 4322 is separate from the anode contacts of the pacemaker device 102, the electrical connection 4328 may be connected directly to the anode contacts of the pacemaker device 102 instead of the isolation seal 4322.

In the present implementation, the connection between each pacemaker device 102 and each connector 2904 is bipolar, where the anode contact of each pacemaker device 102 is electrically connected to the anode contact of each connector 2904. In addition, the isolation seal 4322, caps 4332 of bipolar adaptor 4300, and the distances D enable the bipolar connection. In particular, after each pacemaker device 102 is received into each housing 4302 and 4306, the caps 4332 may seal, via the openings 4310 and 4312, each housing 4302 and 4306. Furthermore, the distance D enables separation of each pacemaker device 102 from the caps 4332, thereby enhancing isolation of each pacemaker device 102 from the exterior of the bipolar adaptor 4300. As described above, the isolation seal 4322 is configured to create electrical isolation between the anode and the cathode contacts of each pacemaker device 102 and each connector 2904 and create electrical isolation between the anode of each pacemaker device 102 and the exterior of the adaptor 4300.

In this implementation, as a redundancy to ensure communication between the pacemaker devices 102 (e.g., implant-to-implant ("i2i") communications), the bipolar adaptor 4300 includes electrical connections 4334 (e.g., electrical traces, vias, or other electrical connections) to connect the cathode contact of one pacemaker device 102 to the anode contact of the other pacemaker device 102 via the isolation seals 4322 and the contacts 4324. In this example, one end of each electrical connection 4334 is connected to each respective isolation seal 4322. The other end of each electrical connection 4334 is connected to each respective contact 4324. As described above, each contact 4324 is in contact with the header assembly 110 of each pacemaker device 102. In examples where the isolation seal 4322 is separate from the anode of the pacemaker device 102, the electrical connection 4334 may be connected directly to the anode of the pacemaker device 102 instead of the isolation seal 4322.

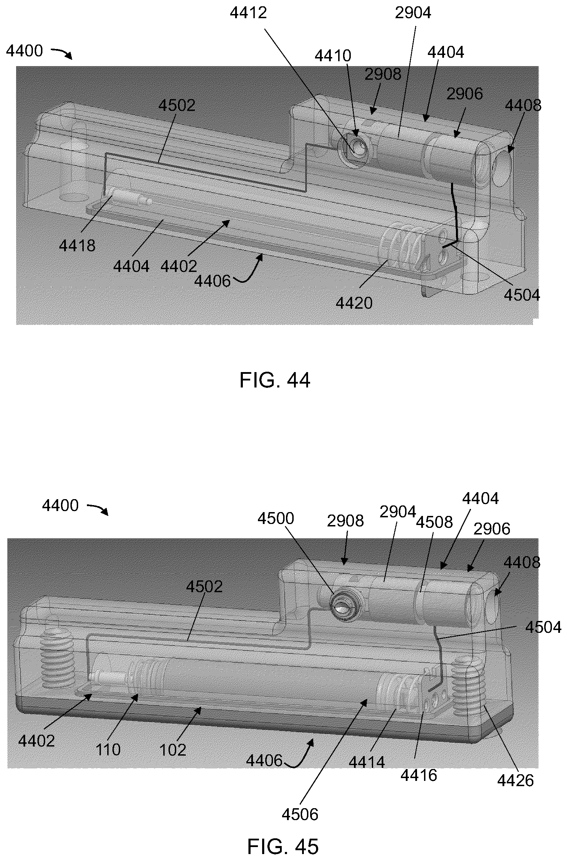

FIGS. 44 and 45 illustrate perspective views of an example of an adaptor 4400 configured to receive the pacemaker device 102 and the connector 2904. The adaptor 4400 includes a first housing 4402 and a second housing 4404. The adaptor 4400 may be made of a material such as a resin, epoxy, and/or other similar materials. The first housing 4402 is configured to receive the pacemaker device 102 through an opening 4406 of the first housing 4402. The second housing 4404 includes an opening 4408 that enables the second housing 4404 to receive the connector 2904. The opening 4408 also enables the connector 2904 to receive a lead (e.g., the lead 424 shown in FIG. 3 or any conventional pacing lead) at a proximal end 2906 of the connector 2904. As shown in FIG. 44, the second housing 4404 also includes an opening 4410 that provides access to a lead lock 4412 of the connector 2904 via a tool slot (not shown) of the lead lock 4412 to receive a tool (e.g., screwdriver). The lead lock 4412 is used to releasably retain the lead in the connector 2904. As shown in FIG. 45, the lead lock 4412 may be sealed by a septum 3500 (similar to septum 3314 shown in FIG. 33) that may be screwed into the lead lock 4412. Other methods of retaining the lead may be used as known by those with ordinary skill in the art.

Additionally, as shown in FIG. 45, the first housing 4402 includes a removable panel (e.g., a gasket) 4414 that seals the opening 4406, and a spring-loaded contact 4416, coil spring 4420, and a contact 4418. The pacemaker device 102 may be inserted laterally into first housing 4400 through opening 4406. Once the pacemaker device 102 is inserted, the removable panel 4414 is sealed into opening 4406 via screws 4426.

In this example, the pacemaker device 102 is electrically connected to the connector 2904 via the contact 4418 of the first housing 4402 and an electrical connection 4502 similar to the electrical connection 2910 shown in FIG. 29B. The pacemaker device 102 is also electrically connected to the connector 2904 via the spring-loaded contact 4416 and an electrical connection 4504 similar to the electrical connection 2912 shown in FIG. 29B. Electrical connections 4502 and 4504 may include electrical traces, vias, or other electrical connectors. The contact 4418 is configured to connect with the header assembly 110 (e.g., an electrode, a helical screw, tines, or other mechanisms positioned in the header assembly 110) of the pacemaker device 102, and to connect with one end of the electrical connection 4502. The other end of the electrical connection 4502 is connected to the distal end 2908 of the connector 2904.

In this example, the spring-loaded contact 4416 is configured to connect with a proximal end 4506 (similar to proximal end 408 shown in FIG. 3) of the pacemaker device 102, and to connect with one end of the electrical connection 4504. The other end of the electrical connection 4504 is connected to a body 4508 (similar to the body 2104 shown in FIG. 21) of the connector 2904. The electrical connection 4504 may act as an anode when the adaptor 4400 is in a bipolar configuration, such as shown in FIG. 45, where the pacemaker device 102 is fully enclosed in the adaptor 4400. In contrast, the adaptor in FIG. 44 is in a unipolar configuration as the pacemaker device 102 would be exposed (the removable panel 4414 sealing the adaptor 4400 is not present), making the pacemaker device act as the anode. The electrical connections 4504 and 4506 enables signals to travel, via the connector 2904, between the pacemaker device 102 and the lead connected to the proximal end 2906 of the connector 2904.

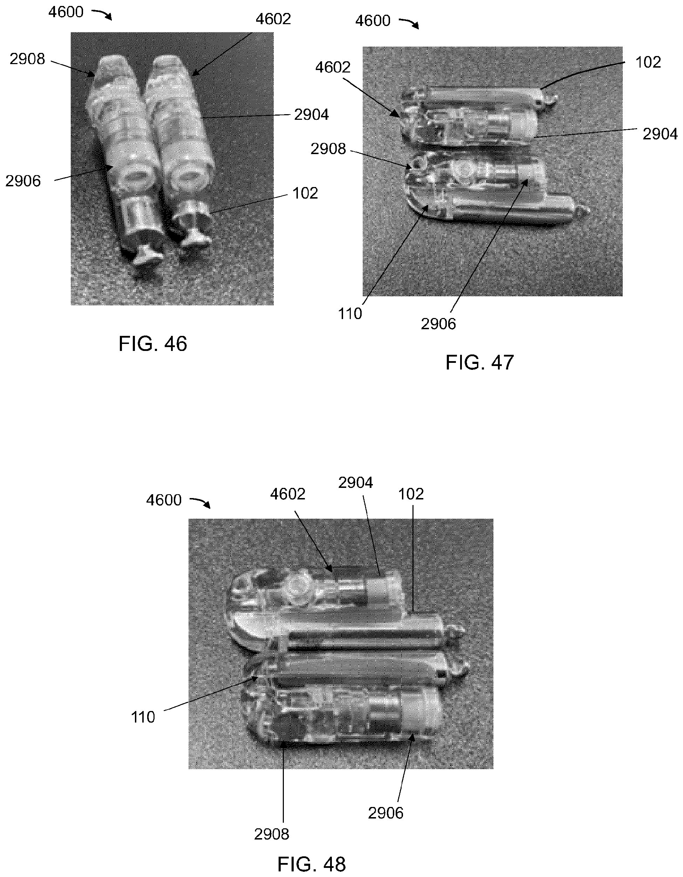

FIGS. 46-48 illustrate a perspective view, a side view and a magnified side view, respectively, of an example of two device assemblies 4600, each including the pacemaker device 102, the connector 2904, and a housing assembly 4602 (e.g., a cylindrical tube and similar to housing assembling 3002 shown in FIG. 30) configured to enclose the connector 2904 (e.g., similar to connector 2102 shown in FIG. 21 or other suitable ISO 5841 IS-1 connectors) and partially enclose the pacemaker device 102. The housing assembly 4602 may be of a material such as a resin, epoxy, and/or other similar materials. The connector 2904 is electrically connected to the pacemaker device 102 through an electrical connection (e.g., similar to electrical connection 2910 shown in FIG. 29B). The electrical connection connects at the header assembly 110 (e.g., an electrode, a helical screw, tines, or other mechanisms positioned in the header assembly 110) of the pacemaker device 102 to the distal end 2908 of the connector 2904. The electrical connection enables signals to travel, via the connector 2904, between the pacemaker device 102 and a lead (e.g., the lead 3202 shown in FIG. 32 or any conventional pacing lead) connected to the proximal end 2906 of the connector 2904. In this example, the electrical connection is unipolar.

### G. Additional Example of a Pacemaker Device Assembly

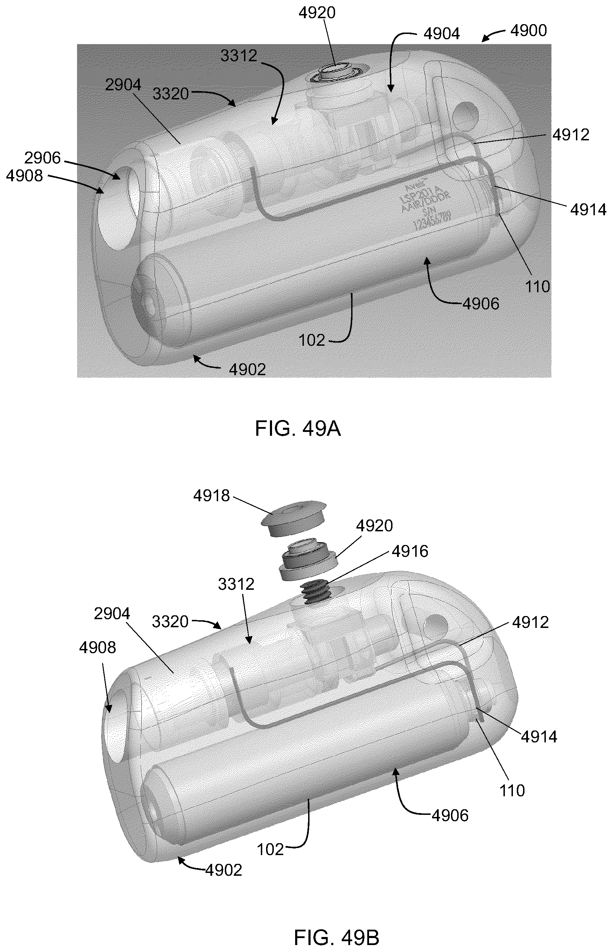

FIGS. 49A and 49B illustrate a perspective view of an example of a bipolar device assembly 4900 including the connector 2904, the pacemaker device 102, and a housing assembly 4902 enclosing the connector 2904 and pacemaker device 102. The housing assembly 4902 includes a first housing 4904 and a second housing 4906. The first housing 4902 is configured to enclose the pacemaker device 102. The second housing 4906 is configured to enclose the connector 2904. The second housing 4906 includes an opening 4908 that enables the second housing 4906 to receive a lead (e.g., the lead 3202 shown in FIG. 32 or any conventional pacing lead) at the proximal end 2906 of the connector 2904. The second housing 4906 also includes an opening, which is sealed by a septum 4920 (similar to the septum 3314 shown in FIG. 33). The opening provides access to a lead lock (not shown) of the connector 2904 via a tool slot (not shown) of the lead lock to receive a tool (e.g., screwdriver). The lead lock is used to releasably retain the lead in the connector 2904 through the proximal end 2906 of the connector 2904.

As shown in FIG. 49B, the septum 4920 may include a set screw 4916 that is operable to releasably retain the lead in the connector 2904 through the proximal end 2906 of the connector 2904 via a tool (e.g., hex key). The septum 4920 may further include a seal 4918 adhered to septum 4920 to prevent dislodgement and hermetically seal the connector 2904 and the adaptor 4400. Other methods of retaining the lead may be used as known by those with ordinary skill in the art.

In this example, the connector 2904 is electrically connected to the pacemaker device 102 through electrical connections 4912 and 4914 (e.g., electrical traces, vias, or other electrical connections). The electrical connection 4912 is similar to the electrical connection 2910 (shown in FIG. 29) and connects the header assembly 110 (e.g., an electrode, a helical screw, tines, or other mechanisms positioned in the header assembly 110) of the pacemaker device 102 to the distal end 2908 of the connector 2904. The electrical connection 4914 is similar to the electrical connection 2912 (shown in FIG. 29) and connects the header assembly 110 of the pacemaker device 102 to an anode contact that may act as the anode of the bipolar device assembly 4900. The electrical connections 4912 and 4914 enable signals to travel, via the connector 2904, between the pacemaker device 102 and the lead connected to the proximal end 2906 of the connector 2904.

The pacemaker devices described herein may include antennas or communication devices as understood in the art to enable the pacemaker devices to communicate with each other and/or with external computer systems using wireless communication (e.g., Bluetooth^{®}). Additionally, the device assemblies and adaptors as described above may be configured to enclose/receive pacemaker devices that are operable through additional feedthroughs and electrical communications connections.

The pacemaker devices, such as those described above, are typically a single use system, designed to be removable and replaceable upon depletion of the battery. This may be suitable for patients transitioning to traditional transvenous devices or leadless devices; however, this may not be suitable for patients requiring permanent pacing due to lack of transvenous access. For such patients, it may be desirable to separate the pacemaker portion from the lead for generator change (i.e., device changeout). To facilitate this option, in some examples, a housing assembly includes a plurality of housings a first housing configured to receive a leadless pacemaker and a second housing operable to receive an ISO 5841 IS-1 connector. Such connectors are suitably capable of receiving conventional pacing leads, such as ISO 5841 IS-1 pacing leads. Another embodiment of this disclosure is to enable the separate pacemaker portions to be swapped with multiple model variations of leadless pacemakers, adaptable for changes in size and future improvements.

The connector, such as an ISO 5841 IS-1 connector, and the second housing may be configured in unipolar or bipolar. In some examples, the device assembly or adaptor may be configured in a single chamber configuration which stimulate either a patient's atrium or ventricle. In another example, the device assembly or adaptor may be configured in a dual chamber configuration to stimulate both the patient's atrium and ventricle by converting two leadless pacemakers. In these examples, the pacing lead can be installed using the techniques described herein or using conventional techniques.

An aspect of the invention relates to a cardiac pacemaker device 400. The cardiac pacemaker device 400 comprises a pacemaker portion 402 including a housing 406, and a distal extension 404 coupled to the housing 406. The housing 406 defines a cavity 458 for containing pacing electronics and battery material. The distal extension 404 comprises a lead 424 electrically connectable with the pacing electronics and extending distally from the housing 406 to a distal end 440 of the distal extension 404. The distal extension 404 also comprises a fixation element 448 positioned at the distal end 440 of the distal extension 404. The fixation element 448 is configured for installation within an epicardium 153 of a heart 152. The distal extension 404 also comprises an electrode 422 electrically connectable with the lead 424 and configured to sense and/or deliver electrical signals at the heart 153.

In an embodiment, the fixation element 448 is an active fixation element 448 that is operable as the electrode 422.

In an embodiment, the electrode 422 is a first electrode 422 and the distal extension 404 further comprises a second electrode 460 that is co-operable with the first electrode 422 as a bipolar electrode pair.

In an embodiment, the second electrode is the active fixation element 448 positioned at the distal end 440 of the distal extension 404 or the second electrode 460 is spaced an axial distance from and located proximally relative to the first electrode 420.

In an embodiment, the fixation element 448 is a helical screw.

In an embodiment, the fixation element 448 is a passive element.

In an embodiment, the fixation element 448 is made from a biocompatible mesh material.

In an embodiment, the lead 424 extends distally from the housing a usable length less than 50 centimeters.

In an embodiment, the pacemaker portion 402 is sized and shaped for implanting at a site proximate to the heart 152.

Another aspect of the invention relates to a pacemaker system 150 comprising a housing assembly 3316. The housing assembly 3316 comprises a first housing 3302 comprising pacing electronics and battery material, a second housing 3304 defining a cavity sized suitably to receive a connector 2904, a fixation element 3312, and electrical connections 2910 operable to connect the pacing electronics and the battery material and the connector 2904.

In an embodiment, the housing assembly 3316 is made from a non-conductive material.

In an embodiment, the first housing 3302 comprises a pacemaker device 102, preferably a leadless pacemaker device 102, in connection with the fixation element 3312.

In an embodiment, the first housing 3302 comprises a set bore 3718 operable to receive a set screw accessed via a tool slot to releasably retain the pacemaker device 102 in the first housing 3302, and the connector 2904 of the second housing 3304 comprises a lead lock 3310 accessed via a tool slot and used to releasably retain a lead 3202 in the connector 2904. The connector 2904 comprises an IS-1 connector.

Another aspect of the invention relates to a cardiac pacemaker system 150 comprising a housing assembly 4300. The housing assembly 4300 comprises a first housing 4302 defining a first cavity and third housing 4306 defining a third cavity. The first housing 4302, such as the first cavity, and the third housing 4306, such as the third cavity, are each sized suitably to receive pacing electronics and battery material. The housing assembly 4300 also comprises a second housing 4304 defining a second cavity and a fourth housing 4308 defining a fourth cavity. The second housing 4304, such as the second cavity, and the fourth housing 4308, such as the fourth cavity, are each sized suitably to receive an electrical connector 4904. The housing assembly 4300 also comprises a fixation element and electrical connections 4326, 4328 operably to connect the first housing 4302 and the second housing 4304.

In an embodiment, the housing assembly 4300 is made from a non-conductive material.

In an embodiment, the first housing 4302 and the third housing 4306 each comprise a pacemaker device 102 in connection with the fixation element.

In an embodiment, the second housing 4304 and the fourth housing 4308 comprise the electrical connections 4326, 4328 including an IS-1 connector, and a lead 3202 electrically connectable with the pacing electronics.

In an embodiment, the lead 3202 comprises an electrode 422 electrically coupleable to the lead 3202 and configured to sense and/or deliver electrical signals at a heart 152.

It should be understood that the various control units, computer systems and the like described herein may include conventional processing apparatus known in the art (*i.e.,* both hardware and/or software), including the capability of executing preprogrammed instructions stored in an associated memory, all performing in accordance with the functionality described herein. It is contemplated that the methods described herein, including without limitation the method steps of embodiments of the invention, may be programmed in a preferred embodiment, with the resulting software being stored in an associated memory and may also constitute the means for performing such methods. Implementation of embodiments, in software, in view of the foregoing enabling description, would require no more than routine application of programming skills by one of ordinary skill in the art. The system may further be of the type having both ROM, RAM, a combination of non-volatile and volatile (modifiable) memory so that the software can be stored and yet allow storage and processing of dynamically produced data and/or signals. Moreover, an article of manufacture in accordance with embodiments of the invention includes a computer-readable storage medium having a computer program encoded thereon for performing the methods described in this application. The computer program includes code that, when executed by a computer, causes the computer to perform the steps of the methods described herein.

Although numerous embodiments of this invention have been described above with a certain degree of particularity, those skilled in the art could make numerous alterations to the disclosed embodiments without departing from the spirit or scope of this invention. All directional references (e.g., plus, minus, upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the scope of the invention as defined in the appended claims.

As for additional details pertinent to the present disclosure, materials and manufacturing techniques may be employed as within the level of those with skill in the relevant art. The same may hold true with respect to method-based aspects of the disclosure in terms of additional acts commonly or logically employed. Also, it is contemplated that any optional feature of the variations described may be set forth and claimed independently, or in combination with any one or more of the features described herein. Likewise, reference to a singular item, includes the possibility that there are plural of the same items present. More specifically, as used herein and in the appended claims, the singular forms "a," "and," "said," and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Unless defined otherwise herein, all technical and scientific terms used herein have the same meaning as commonly understood by one of ordinary skill in the art to which this disclosure belongs.

## Claims

1. A cardiac pacemaker device (400) comprising:
a pacemaker portion (402) comprising a housing (406) defining a cavity (458) for containing pacing electronics and battery material; and
a distal extension (404) coupled to the housing (406), the distal extension (404) comprising:
a lead (424) electrically connectable with the pacing electronics and extending distally from the housing (406) to a distal end (440) of the distal extension (404);
a fixation element (448) positioned at the distal end (440) of the distal extension (404), the fixation element (422) being configured for installation within an epicardium (153) of a heart (152); and
an electrode (422) electrically connectable with the lead (424) and configured to sense and/or deliver electrical signals at the heart (152).

2. The cardiac pacemaker device of claim 1, wherein the fixation element (448) is an active fixation element (448) that is operable as the electrode (422).

3. The cardiac pacemaker device of claim 2, wherein the electrode (422) is a first electrode (422), and wherein the distal extension (404) further comprises a second electrode (460) that is co-operable with the first electrode (422) as a bipolar electrode pair.

4. The cardiac pacemaker device of claim 3, wherein
the second electrode (460) is the active fixation element (448) positioned at the distal end (440) of the distal extension (404); or
the second electrode (460) is spaced an axial distance from and located proximally relative to the first electrode (420).

5. The cardiac pacemaker device of any one of claims 2 to 4, wherein the fixation element (448) is a helical screw.

6. The cardiac pacemaker device of claim 1, wherein the fixation element (448) is a passive element.

7. The cardiac pacemaker device of claim 7, wherein the fixation element (448) is made from a biocompatible mesh material.

8. The cardiac pacemaker device of any one of the preceding claims, wherein
the lead (424) extends distally from the housing (424) a usable length less than 50 centimeters; and/or
the pacemaker portion (402) is sized and shaped for implanting at a site proximate to the heart (152).

9. A cardiac pacemaker system (150) comprising:
a housing assembly (3316) comprising:
a first housing (3302) comprising pacing electronics and battery material;
a second housing (3304) defining a cavity sized suitably to receive a connector (2904);
a fixation element (3312); and
electrical connections (2910) operable to connect the pacing electronic and the battery material and the connector (2904).

10. The cardiac pacemaker system of claim 9, wherein the housing assembly (3316) is made from a non-conductive material.

11. The cardiac pacemaker system of any one of claims 9 and 10, wherein the first housing (3302) comprises a pacemaker device (102) in connection with the fixation element (3312).

12. The cardiac pacemaker system of claim 11, wherein
the first housing (3302) comprises a set bore (3718) operable to receive a set screw accessed via a tool slot to releasably retain the pacemaker device (102) in the first housing (3302);
the connector (2904) of the second housing (3304) comprises a lead lock (3310) accessed via a tool slot and used to releasably retain a lead (3202) in the connector (2904); and
the connector (2904) comprises an IS-1 connector.

13. A cardiac pacemaker system (150) comprising:
a housing assembly (4300) comprising:
a first housing (4302) defining a first cavity and a third housing (4306) defining a third cavity, wherein the first housing (4302) and the third housing (4306) are each sized suitably to receive pacing electronics and battery material;
a second housing (4304) defining a second cavity and a fourth housing (4308) defining a fourth cavity, wherein the second housing (4304) and the fourth housing (4308) are sized suitably to receive an electrical connector (4804);
a fixation element; and
electrical connections (4326, 4328) operable to connect the first housing (4302) and the second housing (4304).

14. The cardiac pacemaker system of claim 15, wherein
the housing assembly (4300) is made from a non-conductive material; and/or
the first housing (4302) and the third housing (4306) each comprise a pacemaker device (102) in connection with the fixation element; and/or
the second housing (4304) and the fourth housing (4308) comprise the electrical connections (4326, 4328) including an IS-1 connector, and a lead (3202) electrically connectable with the pacing electronics.

15. The cardiac pacemaker system of claim 14, wherein the lead (3202) comprises an electrode (422) electrically coupleable to the lead (3202) and configured to sense and/or deliver electrical signals at a heart (152).
